# EUROPEAN PATENT APPLICATION

(11) **EP 4 545 557 A1**
(43) Date of publication of application: **30.04.2025**
(21) Application number: 23919260.2
(22) Date of filing: 29.06.2023
(51) Int. Cl.: C07K 14/705, C12N 15/12, C07K 19/00, C12N 15/62, C12N 15/867, A61K 39/00, C07K 16/30, A61P 35/00, A61P 35/02, G01N 33/50, G01N 33/68, C07K 14/755

(54) **SYNNOTCH RECEPTOR AND USE THEREOF**

(30) Priority: 31.01.2023 CN 202310093577
(71) Applicant: SHANGHAI NK CELLTECH CO., LTD., Shanghai 201318 (CN)
(72) Inventor: WANG, Ting, Shanghai 201318 (CN); ZHANG, Cai, Shanghai 201318 (CN); ZHOU, Yingli, Shanghai 201318 (CN); CHEN, Minhua, Shanghai 201318 (CN); XIE, Siqi, Shanghai 201318 (CN)
(74) Representative: Dehns Germany Partnerschaft mbB
(86) International application number: PCT/CN2023/103857
(87) International publication number: WO 2024/159695

(57) **Abstract**

The present disclosure provides a chimeric polypeptide. The chimeric polypeptide includes: an extracellular region having an activity of binding to a first molecule; a transmembrane region having an N-terminus linked to a C-terminus of the extracellular region; and an intracellular region having an N-terminus linked to a C-terminus of the transmembrane region, wherein the transmembrane region includes an isolated polypeptide having at least 80% identity with a transmembrane region of a Notch receptor protein derived from *Xenopus tropicalis.*

## Description

### FIELD

The present disclosure belongs to the field of biotechnology. Specifically, the present disclosure relates to a SynNotch receptor and a use thereof. More specifically, the present disclosure relates to a use of an isolated polypeptide in the preparation of a SynNotch synthetic receptor, a chimeric polypeptide, a first nucleic acid molecule, a first expression vector, a recombinant cell, a pharmaceutical composition and use thereof, a method for activating an immune cell, and a method for tracking a first cell-second cell contact.

### BACKGROUND

With the development of synthetic biology, it has become a reality to modify the cells to enable them recognize specific extracellular signals, transmit the recognized signals within the modified cells and make an artificially designed response, and has gradually been applied in the biomedical field. For example, the application of chimeric antigen receptor (CAR) technology in clinical treatment. In addition, in 2016, Wend ell A. Lim's team developed a SynNotch receptor system based on a Notch signaling pathway. This system can also recognize the on cell membrane surface ligands, and signal output thereof is not limited to simple immune cell activation signals, which can realize many different signal output types, such as activation and inhibition of specific gene expression. This receptor system can realize a variety of combinations of different input signals and output signals, and a plurality groups of systems within the same system (cell) are orthogonal; at the same time, the system may operate in many cells and is universal; and the system also has many excellent characteristics such as simplicity and controllability. Therefore, the system is a powerful tool for applying synthetic biology to modify cells.

In biology, a traditional Notch signaling pathway (system) includes Notch-corresponding ligands, Notch molecules, and Notch-regulated downstream genes. The Notch molecule is a transmembrane molecule, which can be divided into three parts: extracellular domain, transmembrane domain, and intracellular domain. After the extracellular domain of the Notch molecule binds to the corresponding Notch ligand, the cell where the ligand is located undergoes endocytosis, pulling the Notch molecule, exposing the S2 protease cleavage site on the extracellular domain of the Notch molecule, and the Notch molecule is then cleaved by members of the metalloprotease ADAMs family. Furthermore, along with the cleavage and detachment of the extracellular domain, the intracellular domain of the Notch molecule is cleaved by γ-secretase and released into the cell, and then enters the nucleus as a transcription factor to regulate the expression of downstream related genes. Wend ell A. Lim's team modified the murine Notch1 receptor by replacing its extracellular domain with a single chain antibody or nanoantibody, and replacing its the intracellular domain with a transcriptional activation or transcriptional inhibition domain, retaining only the transmembrane domain that can be recognized and cleaved by proteases, and also equipping with downstream regulated gene elements. For different targets, the extracellular domain adopts a single chain antibody that specifically recognizes the antigen, while the intracellular domain regulates the expression of pre-set target genes or factors. Gene regulatory circuits initiated by different antigens may be designed in the same cell with good orthogonality. The SynNotch system may be applied to many cell types, including neural cells, tumor cells, epidermal cells, and immune cells. The combined application of CAR technology and SynNotch system in immune cell modification can achieve the "AND gate" activation of immune cells, that is, the immune cells can only be activated when the cells express two specific surface antigens at the same time. By changing the genetic elements regulated downstream of SynNotch, immune cells can secrete single chain antibodies, cytokines, and other therapeutic factors after contacting with specific antigens, and have anti-tumor effects both in vitro and in vivo. It can be seen that the SynNotch receptor system has great technical advantages in cell modification.

### SUMMARY

The present disclosure aims to solve, at least to some extent, at least one of the technical problems existing in the prior art. To this end, the present disclosure provides a SynNotch receptor, which can enhance its ability to activate downstream genes and reduce basal leakage activation levels.

The present disclosure is based on the following findings of the inventors:

The SynNotch system is very powerful, but the currently commonly used initial version of the SynNotch system (also known as SynNotch synthetic receptor, SynNotch) has the following disadvantages. First, after the initial version of SynNotch is activated, its downstream transcriptional expression regulation ability is not very strong, that is, the expression level of activated downstream genes is not very high, which will limit the use of SynNotch, in particular for situations where downstream genes need to have a higher expression level to be effective. Second, the initial version of SynNotch has a high basal leakage level. For the downstream genes that require strict regulation, the high level of leakage expression may lead to the failure of the SynNotch molecular switch, and the molecular expression circuit will directly bypass the SynNotch molecular switch to achieve expression.

However, the inventors unexpectedly discovered through experiments that the key point of the above problem lies in the deficiency of the transmembrane domain of the SynNotch molecule. The initial version of the SynNotch synthetic receptor selected the transmembrane domain of the mouse-derived Notch molecule as the transmembrane domain of the SynNotch synthetic receptor. Considering the differences in the S2 and S3 cleavage sequences carried on the Notch transmembrane domains from different species, as well as the conformational and sequence preferences of S2 and S3 cleavage enzymes from different species, the activation efficiency and leakage efficiency of SynNotch synthetic receptors constructed using different transmembrane domains are bound to be different in the cells of different species. With the ultimate goal of modifying human cells using SynNotch synthetic receptors, the inventors unexpectedly found during the experiment that selecting the Notch transmembrane domain of *Xenopus tropicalis* can improve the ability and efficiency of SynNotch synthetic receptors for transcription and activation of downstream genes, and reduce the basal leakage activation of SynNotch synthetic receptors, so that the SynNotch synthetic receptors can play a more effective role.

Therefore, in one aspect of the present disclosure, the present disclosure provides a use of an isolated polypeptide in the preparation of a SynNotch synthetic receptor, wherein the isolated polypeptide has at least 80% identity with a transmembrane region of a Notch receptor protein derived from *Xenopus tropicalis.* The inventors found that the SynNotch synthetic receptor prepared by selecting the transmembrane region of the Notch receptor protein of *Xenopus tropicalis* can improve its ability and efficiency for transcription and activation of downstream genes and reduce the basal leakage of the SynNotch synthetic receptor.

In another aspect of the present disclosure, the present disclosure provides a chimeric polypeptide. According to an embodiment of the present disclosure, the chimeric polypeptide includes: an extracellular region having an activity of binding to a first molecule; a transmembrane region, an N-terminus of the transmembrane region being linked to a C-terminus of the extracellular region; and an intracellular region, an N-terminus of the intracellular region being linked to a C-terminus of the transmembrane region, wherein the transmembrane region includes an isolated polypeptide, and the isolated polypeptide has at least 80% identity with a transmembrane region of a Notch receptor protein derived from *Xenopus tropicalis.* In the chimeric polypeptide according to the present disclosure, the transmembrane region of the Notch receptor protein of *Xenopus tropicalis* is selected as the transmembrane region of the chimeric polypeptide (i.e., the SynNotch synthetic receptor), which can improve the ability and efficiency of the chimeric polypeptide for transcription and activation of downstream genes and reduce the basal leakage of the chimeric polypeptide.

In yet another aspect of the present disclosure, the present disclosure provides a first nucleic acid molecule. According to an embodiment of the present disclosure, the first nucleic acid molecule encodes the above-mentioned chimeric polypeptide. The first nucleic acid molecule according to an embodiment of the present disclosure encodes the above-mentioned chimeric polypeptide.

In yet another aspect of the present disclosure, the present disclosure provides a first expression vector. According to an embodiment of the present disclosure, the first expression vector carries the above-mentioned first nucleic acid molecule. After the first expression vector according to the present disclosure is introduced into a suitable recipient cell, the above-mentioned chimeric polypeptide can be expressed.

In yet another aspect of the present disclosure, the present disclosure provides a recombinant cell. According to an embodiment of the present disclosure, the recombinant cell carries the above-mentioned first nucleic acid molecule or the above-mentioned first expression vector; or expresses the above-mentioned chimeric polypeptide. The recombinant cell according to the present disclosure can effectively express the above-mentioned chimeric polypeptide.

In yet another aspect of the present disclosure, the present disclosure provides a pharmaceutical composition. According to an embodiment of the present disclosure, the pharmaceutical composition includes the above-mentioned chimeric polypeptide, the above-mentioned first nucleic acid molecule, the above-mentioned first expression vector, or the above-mentioned recombinant cell. As can be seen from the foregoing, the cell expressing the above-mentioned chimeric polypeptide can recognize the first molecule, and after binding to the first molecule, the cells can realize many different signal output types, such as activation and inhibition of specific gene expression. Alternatively, the immune cell expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. Therefore, the pharmaceutical composition containing the above-mentioned chimeric polypeptide, first nucleic acid molecule, first expression vector, or recombinant cell can target the first molecule for preventing and/or treating related diseases, such as for treating cancer.

In yet another aspect of the present disclosure, the present disclosure provides a use of the above-mentioned chimeric polypeptide, the above-mentioned first nucleic acid molecule, the above-mentioned first expression vector, the above-mentioned recombinant cell, or the above-mentioned pharmaceutical composition in the preparation of a medicament for preventing and/or treating a disease. As can be seen from the foregoing, the cell expressing the above-mentioned chimeric polypeptide can recognize the first molecule, and after binding to the first molecule, the cell can realize many different signal output types, such as activation and inhibition of specific gene expression. Alternatively, the immune cell expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. In addition, the pharmaceutical composition includes the above-mentioned chimeric polypeptide, the first nucleic acid molecule, the first expression vector, or the recombinant cell. Therefore, the medicament containing the above-mentioned chimeric polypeptide, first nucleic acid molecule, first expression vector, recombinant cell, or pharmaceutical composition can target the first molecule for preventing and/or treating related diseases, such as for treating cancer.

In another aspect of the present disclosure, the present disclosure provides the above-mentioned chimeric polypeptide, the above-mentioned first nucleic acid molecule, the above-mentioned first expression vector, the above-mentioned recombinant cell, or the above-mentioned pharmaceutical composition for use in the prevention and/or treatment of a disease. As can be seen from the foregoing, the cell expressing the above-mentioned chimeric polypeptide can recognize the first molecule, and after binding to the first molecule, the cell can realize many different signal output types, such as activation and inhibition of specific gene expression. Alternatively, the immune cell expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. In addition, the pharmaceutical composition includes the above-mentioned chimeric polypeptide, the first nucleic acid molecule, the first expression vector, or the recombinant cell. Therefore, the medicament containing the above-mentioned chimeric polypeptide, first nucleic acid molecule, first expression vector, recombinant cell, or pharmaceutical composition can target the first molecule for preventing and/or treating related diseases, such as cancer.

In yet another aspect of the present disclosure, the present disclosure provides a method for treating or preventing a disease. According to an embodiment of the present disclosure, the method includes: administering a pharmaceutically acceptable dose of the above-mentioned recombinant cell or the above-mentioned pharmaceutical composition to a subject. As can be seen from the foregoing, the cell expressing the above-mentioned chimeric polypeptide can recognize the first molecule, and after binding to the first molecule, the cell can realize many different signal output types, such as activation and inhibition of specific gene expression. Alternatively, the immune cell expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. In addition, the pharmaceutical composition includes the above-mentioned chimeric polypeptide, the first nucleic acid molecule, the first expression vector, or the recombinant cell. Therefore, the medicament containing the recombinant cell or the pharmaceutical composition which includes the chimeric polypeptide can target the first molecule for preventing and/or treating related diseases, such as for treating cancer.

In yet another aspect of the present disclosure, the present disclosure provides a method for activating an immune cell. According to an embodiment of the present disclosure, the method includes: performing a first contact on the immune cell and a first molecule, wherein the immune cell expresses the above-mentioned chimeric polypeptide. Therefore, the immune cell expressing the above-mentioned chimeric polypeptide can recognize the first molecule, and the immune cell can be activated after binding to the first molecule.

In yet another aspect of the present disclosure, the present disclosure provides a method for tracking a first cell-second cell contact. According to an embodiment of the present disclosure, the method includes: performing a second contact on the first cell and the second cell, wherein the first cell expresses the above-mentioned chimeric polypeptide and a reporter gene protein, the intracellular region of the chimeric polypeptide being a transcription activator protein, 5'-end of a third nucleic acid molecule encoding the reporter gene protein being linked to an inducible expression nucleic acid sequence for binding to the transcription activator protein, and the second cell expresses a first molecule; and determining the contact situation between the first cell and the second cell based on a detection result of the reporter gene protein in the first cell. Therefore, the above-mentioned method can be used to determine whether the first cell is in contact with the second cell based on the detection result of the reporter gene protein.

Additional aspects and advantages of the present disclosure will be provided in part in the following description, or will become apparent in part from the following description, or can be learned from practicing of the present disclosure.

### BRIEF DESCRIPTION OF THE DRAWINGS

The foregoing and/or additional aspects and advantages of the present disclosure will become apparent and readily appreciated from the following description of the embodiments in conjunction with the accompanying drawings of which:
FIG 1 is a schematic diagram of the structure of the SynNotch synthetic receptor in Example 1 according to the present disclosure;
FIG 2 is a schematic diagram of the SynNotch synthetic receptor cell recognizing a specific extracellular signal in Example 2 according to the present disclosure;
FIG 3 is a schematic diagram of the structure of the membrane-anchored GFP in Example 2 according to the present disclosure;
FIG 4 illustrates leakage efficiency and effective activation efficiency of the SynNotch synthetic receptor containing the Notch transmembrane domain of mouse and the SynNotch synthetic receptor containing the Notch transmembrane domain of *Xenopus tropicalis* in Example 2 according to the present disclosure;
FIG 5 is a schematic diagram of the structure of the membrane-anchored CD19 in Example 3 according to the present disclosure;
FIG 6 illustrates the activation results of SynNotch-CAR-Jurkat T cells by target cancer cells in Example 3 according to the present disclosure; and
FIG 7 illustrates the killing efficiency of SynNotch-CAR-NK cells on target cancer cells in Example 4 according to the present disclosure.

### DETAILED DESCRIPTION

Embodiments of the present disclosure are described in detail below. The embodiments described below are exemplary and are only used to illustrate the present disclosure, and are not to be construed as limiting the present disclosure.

It should be noted that the terms "first" and "second" are used for descriptive purposes only and should not be construed as indicating or implying relative importance or implicitly indicating the number of technical features indicated. Thus, a feature defined as "first" or "second" may explicitly or implicitly include one, two, or more such features. Further, in the description of the present disclosure, the meaning of "plurality" is two or more, unless otherwise specified.

As used herein, the terms "include" or "including" are open expressions, that is, including the contents specified in the present disclosure but not excluding other contents.

As used herein, the terms "optionally", "optional", or "optionally" generally mean that the subsequently described event or condition may, but does not necessarily occur, and the description includes situations in which the event or condition occurs, as well as situations in which the event or condition does not occur.

As used herein, the terms "identity", "homology", or "similarity" are all used to describe the percentage of identical amino acids or nucleotides between two amino acid sequences or nucleic acid sequences relative to the amino acid sequence or nucleic acid sequence of a reference sequence, determined by conventional methods, for example, see Ausubel et al., eds. (1995), Current Protocols in Molecular Biology, Chapter 19 (Greene Publishing and Wiley-Interscience, New York); and ALIGN program (Dayhoff (1978), Atlas of Protein Sequence and Structure 5: Suppl. 3 (National Biomedical Research Foundation, Washington, DC). There are many algorithms for aligning sequences and determining sequence identity, including homology alignment algorithm of Needleman et al. (1970) J. Mol. Biol. 48:443; local homology algorithm of Smith et al. (1981) Adv. Appl. Math. 2:482; similarity search method of Pearson et al. (1988) Proc. Natl. Acad. Sci. 85:2444; Smith-Waterman algorithm (Meth. Mol. Biol. 70:173-187 (1997); and BLASTP, BLASTN, and BLASTX algorithms (see Altschul et al. (1990) J. Mol. Biol. 215:403-410). Computer programs that utilize these algorithms are also available, and include, but are not limited to, ALIGN or Megalign (DNASTAR) software, or WU-BLAST-2 (Altschul et al., Meth. Enzym., 266:460-480 (1996)); or GAP, BESTFIT, BLAST Altschul et al., supra, FASTA, and TFASTA, available in the Genetics Computing Group (GCG) package, Version 8, Madison, Wisconsin, USA; and CLUSTAL in the PC/Gene program provided by Intelligenetics, Mountain View, California.

As used herein, the term "at least 80% identity" refers to at least 80%, and may be 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9% identity to the respective reference sequence

As used herein, the term "expression vector" generally refers to a nucleic acid molecule that can be inserted into a suitable host for self-replication, which transfers the inserted nucleic acid molecule into the host cell and/or between host cells. The expression vector may include a vector mainly used to insert DNA or RNA into cells, a vector mainly used to replicate DNA or RNA, and a vector mainly used for expression of transcription and/or translation of DNA or RNA. The expression vector also includes vectors having multiple functions as described above. The expression vector may be a polynucleotide that may be transcribed and translated into a polypeptide when introduced into a suitable host cell. Generally, the expression vector can produce the desired expression product by culturing suitable host cells containing the expression vector.

As used herein, the term "recombinant cell" generally refers to a cell having unique stable genetic traits obtained by modifying or recombining the genetic material of a host cell using genetic engineering technology or cell fusion technology. The term "host cell" refers to a prokaryotic cell or a eukaryotic cell into which a recombinant expression vector can be introduced. As used herein, the term "transformed" or "transfected" refers to the introduction of a nucleic acid (e.g., a vector) into a cell by various techniques known in the art. Suitable host cells may be transformed or transfected with the DNA sequences of the present disclosure and may be used for the expression and/or secretion of the target protein.

As used herein, the term "pharmaceutical composition" generally refers to a unit dosage form, and may be prepared by any of the methods well known in the pharmaceutical field. All methods include the step of combining the active ingredient with the carrier which constitutes one or more accessory ingredients. Generally, compositions are prepared by uniformly and sufficiently combining an active chimeric polypeptide or recombinant cells with a liquid carrier, a finely divided solid carrier, or both.

As used herein, the term "pharmaceutically acceptable excipient" may include any solvent, solid excipient, diluent, or other liquid excipient, etc., suitable for a particular target dosage form. Except to the extent that any conventional excipients are incompatible with the chimeric peptides or recombinant cells of the present disclosure, such as producing any adverse biological effects or interacting in a deleterious manner with any other component of the pharmaceutically acceptable composition, their use is also contemplated by the present disclosure.

As used herein, the term "administering" refers to introducing a predetermined amount of a substance into a patient by some suitable means. The recombinant cell or pharmaceutical composition of the present disclosure may be administered by any common route as long as it can reach the intended tissue. Various modes of administration are contemplated, including peritoneal injection, intravenous injection, intramuscular injection, subcutaneous injection, etc., but the present disclosure is not limited to these exemplified modes of administration. Preferably, the composition of the present disclosure is administered by intravenous injection or subcutaneous injection.

As used herein, the term "treat/treatment" refers to a procedure to obtain a desired pharmacological and/or physiological effect. The effect may be prophylactic, in terms of completely or partially preventing the disease or its symptoms, and/or therapeutic, in terms of partially or completely curing the disease and/or adverse effects caused by the disease. As used herein, "treat/treatment" encompasses diseases in mammals, particularly humans, and includes: (a) preventing the occurrence of a disease or condition in an individual who is susceptible to the disease but has not yet been diagnosed with the disease; (b) inhibiting the disease, such as arresting the progression of the disease; or (c) alleviating the disease, such as alleviating the symptoms associated with the disease. As used herein, "treat/treatment" encompasses any administration of a chimeric polypeptide, recombinant cell, pharmaceutical composition, or medicament to an individual to treat, cure, alleviate, ameliorate, mitigate, or inhibit a disease in the individual, including but not limited to administering a medicament containing a chimeric polypeptide, recombinant cell, or pharmaceutical composition described herein to an individual in need thereof.

The present disclosure provides a use of an isolated polypeptide in the preparation of a SynNotch synthetic receptor, a chimeric polypeptide, a first nucleic acid molecule, a first expression vector, a recombinant cell, a pharmaceutical composition and use thereof, a method for activating immune cells, and a method for tracking a first cell-second cell contact, which will be described in detail below.

### Use of an isolated polypeptide in the preparation of a SynNotch synthetic receptor

In one aspect of the present disclosure, the present disclosure provides a use of an isolated polypeptide in the preparation of a SynNotch synthetic receptor. The isolated polypeptide has at least 80% identity with a transmembrane region of a Notch receptor protein derived from *Xenopus tropicalis.* The inventors found that the SynNotch synthetic receptor prepared by selecting the transmembrane region of the Notch receptor protein of *Xenopus tropicalis* can improve its ability and efficiency for transcription and activation of downstream genes and reduce the basal leakage of the SynNotch synthetic receptor.

According to an embodiment of the present disclosure, the isolated polypeptide has 100% identity with the transmembrane region of the Notch receptor protein derived from *Xenopus tropicalis.*

According to an embodiment of the present disclosure, the transmembrane region of the Notch receptor protein derived from *Xenopus tropicalis* has an amino acid sequence as set forth in SEQ ID NO: 1.

ILDYGFIGGLGKNITPPDNEEICENEQCAELADNKICNANCNNHACGWDG
GDCSLNFNDPWKNCTQSLQCWKYFNDGKCDSQCNNSGCLYDGFDCQKVEVQCNPL
YDQYCRDHFQDGHCDQGCNNAECEWDGLDCDNMPENLAEGTLLIVVLMPPEKLKN
NSVNFLRELSRVLHTNVVFKKDSKGEYKIYPYYGNEEELKKHHIKKRSAASWSDAPT
AIFSTMKESVLPGRRRRELDQMEVRGSIVYLEIDNRQCYKSSSQCFTSATDVAAFLGA
LATHGNLNIPYKIEAVKSEIVETAKPPPPLYAMFSMLVIPLLIIFVIMVVIVNKKRRR
(SEQ ID NO: 1).

### Chimeric polypeptide

In another aspect of the present disclosure, the present disclosure provides a chimeric polypeptide. According to an embodiment of the present disclosure, the chimeric polypeptide includes: an extracellular region having an activity of binding to a first molecule; a transmembrane region, an N-terminus of a transmembrane region being linked to a C-terminus of the extracellular region; and an intracellular region, an N-terminus of an intracellular region being linked to a C-terminus of the transmembrane region, wherein the transmembrane region includes an isolated polypeptide, the isolated polypeptide having at least 80% identity with a transmembrane region of a Notch receptor protein derived from *Xenopus tropicalis.*

The inventors found that selecting the transmembrane region of the Notch receptor protein of the *Xenopus tropicalis* as the transmembrane region of the chimeric polypeptide (i.e. the SynNotch synthetic receptor) can improve the ability and efficiency of the chimeric polypeptide for transcription and activation of downstream genes and reduce the basal leakage of the chimeric polypeptide. Furthermore, the cells expressing the chimeric polypeptide can recognize the first molecule, and after binding to the first molecule, the cells can realize many different signal output types, such as activation and inhibition of specific gene expression. Moreover, the chimeric polypeptide can be used to prepare the immune cells expressing the chimeric polypeptide, which can secrete factors with therapeutic effects (such as single chain antibodies, cytokines, etc.) for anti-tumor after contacting the first molecule.

According to an embodiment of the present disclosure, the isolated polypeptide has 100% identity with the transmembrane region of the Notch receptor protein derived from *Xenopus tropicalis.*

According to an embodiment of the present disclosure, the transmembrane region of the Notch receptor protein derived from *Xenopus tropicalis* has an amino acid sequence as set forth in SEQ ID NO: 1.

According to an embodiment of the present disclosure, the transmembrane region further includes an epidermal growth factor-like repeat sequence (EGF repeat) and/or a RAM sequence. Thereby, the basal leakage activation of SynNotch synthetic receptors can be further reduced.

According to an embodiment of the present disclosure, the epidermal growth factor-like repeat sequence has an amino acid sequence as set forth in SEQ ID NO: 15.
VVSPCASRPCYNGGTCQFSPEEPFFQCFCPTNFNGLFCH (SEQ ID NO: 15).

According to an embodiment of the present disclosure, the RAM sequence has an amino acid sequence as set forth in SEQ ID NO: 16.
EHGQLWFP (SEQ ID NO: 16).

According to an embodiment of the present disclosure, a C-terminus of the epidermal growth factor-like repeat sequence is linked to the N-terminus of the isolated polypeptide; and/or, the C-terminus of the isolated polypeptide is linked to an N-terminus of the RAM sequence.

According to an embodiment of the present disclosure, the first molecule includes at least one of a tumor antigen, a virus, a bacterium, an endotoxin, an antibody, a cell receptor, and a ligand of a cell receptor.

As used herein, the term "tumor antigen" generally refers to antigenic substances that emerge newly or are overexpressed during the occurrence and development of tumors. Tumor antigens are classified into tumor-specific antigens and tumor-associated antigens according to their specificity. Tumor-specific antigens (TSA) are new antigens that are unique to tumor cells or only exist in certain tumor cells but not in normal cells. Tumor-associated antigens (TAA) refer to antigens that are not unique to tumor cells and also exist in normal cells and other tissues, but their content increases significantly when the cells become cancerous, including but are not limited to PD-L1, PD-1, TGF-β, CEA, GD2, and GD3.

As used herein, the term "cell receptor" or "receptor" should be understood in a broad sense, and may refer to molecules located on the cell membrane that can recognize and bind to various extracellular signal molecules (ligands), including but not limited to growth factor receptors (such as VEGF receptors), (NKG2D polypeptides (receptors for MICA, MICB, and ULB6), cytokine receptors (such as IL-13 receptors, IL-2 receptors, etc.), epidermal growth factor (EGF) receptors, Her2, CD27, natural cytotoxic receptors (NCR) (such as NKP30 (NCR3/CD337) polypeptides (receptors for HLA-B associated transcript 3 (BAT3) and B7-H6), etc.), T cell antigen receptors, dihydrofolate receptors, chimeric cytokine receptors, Fc receptors, extracellular matrix receptors (such as integrins), cell adhesion receptors (such as cadherins), immunomodulatory receptors (including positive co-receptors (such as CD28) and negative (immunosuppressive) co-receptors (such as PD1)) and receptors for immunomodulatory molecules (such as TGFβ), etc.

As used herein, the term "ligand of cell receptor" should be understood in a broad sense and may refer to chemical substances that can bind to and interact with cell membrane receptors and produce specific biological effects, such as polypeptides, nucleic acids, glycoproteins, small molecules, carbohydrates, lipids, glycolipids, lipoproteins, lipopolysaccharides, etc., including but not limited to cytokines (e.g., IL-13, etc.), growth factors (e.g., heregulin, vascular endothelial growth factor (VEGF)), etc.), peptide hormones, integrin binding peptides (e.g., peptides including the sequence Arg-Gly-Asp), N-glycans, etc.

Exemplarily, the ligand is VEGF and the receptor is VEGF receptor; or the ligand is heregulin and the receptor is Her2.

As used herein, the term "cytokine" should be understood in a broad sense and may refer to a class of proteins or small molecule polypeptides that can transmit information between cells and have immune regulation and effector functions, such as IL-10. "Cytokine receptor" should be understood in a broad sense and may refer to receptors on the cell surface that can bind to cytokines, such as Her2 and IL-10R.

In a preferred embodiment of the present disclosure, the tumor antigen is a tumor-specific antigen.

According to an embodiment of the present disclosure, the first molecule includes at least one of GFP, eGFP, CD19, ALPPL2, BCMA, SIRPα, CD1a, CD1b, CD1c, CD1d, CD1e, CD2, CD3d, CD3e, CD3g, CD4, CD5, CD7, CD8a, CD8b, CD20, CD21, CD22, CD23, CD25, CD27, CD28, CD30, CD33, CD34, CD38, CD40, CD44, CD44v6, CD45, CD48, CD51, CD52, CD56, CD59, CD66, CD70, CD71, CD72, CD73, CD74, CD79A, CD79B, CD80, CD86, CD94, CD95, CD133, CD134, CD140, CD152. CD154, CD158, CD178, CD181, CD182, CD183, CD200, CD210, CD221, CD246, CD252, CD253, CD261, CD262, CD273, CD274, CD276, CD279, CD295, CD339, CD340, EGFR, HER2, FGFR2, AFP, CA125, MSLN, GPC3, CEA, CLDN18.2, EpCAM, PSCA, GD2, IL-13, IL-13RA2, ROR1, MUC 1, PSMA, MAGEA1, 4-1BB, 5T4, BAFF, CA242, CA-IX, MET, CCR4, CNTO888, FAP, MORAb-009, VEGF-A, VEGFR-1, and VEGFR-2.

According to an embodiment of the present disclosure, the extracellular region includes a first binding protein or a fragment thereof that binds to the first molecule.

According to an embodiment of the present disclosure, the first binding protein or a fragment thereof includes at least one of an antibody or a functional fragment thereof, a receptor, a ligand of a receptor, and a cell adhesion molecule.

As used herein, "cell adhesion molecule" or "CAM" may refer to a polypeptide that binds to a component of the extracellular matrix (ECM) or to a cell surface molecule. For example, the cell adhesion molecule may be the extracellular region of CAM, wherein CAM may be a calcium-independent adhesion molecule, for example, CAM is an immunoglobulin superfamily CAM, and CAM may also be a calcium-dependent adhesion molecule, for example, CAM is an integrin, a cadherin, or a selectin; the cell adhesion molecule may be an integrin, for example, a cadherin, such as E-cadherin, P-cadherin, N-cadherin, R-cadherin, M-cadherin, etc.; and the cell adhesion molecule may also be a selectin, such as E-selectin, L-selectin or P-selectin.

As used herein, the term "antibody" is used in the broadest sense and may include full-length monoclonal antibodies, multispecific antibodies, and chimeric antibodies without limitation to the specific structure, as long as they exhibit the desired biological activity. The antibody usually includes a light chain with a lighter molecular weight and a heavy chain with a heavier molecular weight, and the heavy chain (H chain) and the light chain (L chain) are linked by disulfide bonds to form an antibody molecule. Wherein, the amino acid sequence at the amino terminus (N terminus) of the peptide chain varies greatly and is called the variable region (V region); the carboxyl terminus (C terminus) is relatively stable and varies very little, and is called the constant region (C region). The V regions of the L chain and H chain are called VL and VH, respectively.

As used herein, the terms "full-length antibody", "full-length monoclonal antibody", or "full-length monoclonal antibody" are composed of at least two identical light chains and at least two identical heavy chains linked by interchain disulfide bonds, such as immunoglobulin G (IgG), immunoglobulin A (IgA), immunoglobulin M (IgM), immunoglobulin D (IgD), or immunoglobulin E (IgE).

As used herein, the term "functional fragment" refers to a fragment including part or all of an antibody, which lacks at least some of the amino acids present in the full-length chain but is still capable of specifically binding to an antigen. For example, the fragment may include part or all of an antibody CDR. Such fragments are biologically active in that they bind to the antigen and can compete with other antigen-binding molecules (including intact antibodies) for binding to a given epitope. Such fragments are selected from Fab, Fv, scFv, or single domain antibodies. Such fragments may be produced by recombinant nucleic acid technology, or may be produced by enzymatic or chemical cleavage of antigen binding molecules, including intact antibodies.

According to an embodiment of the present disclosure, the first binding protein or a fragment thereof is a monoclonal antibody or a polyclonal antibody that binds to the first molecule.

As used herein, the terms "polyantibody" and "multispecific antibody" are synonymous and both refer to antibodies that can recognize multiple antigenic epitopes, such as antibodies that can recognize two antigenic epitopes (bispecific antibodies, abbreviated as BsAb), antibodies that can recognize three antigenic epitopes, or antibodies that can recognize four antigenic epitopes. This is understood in a broad sense and the specific structure is not limited as long as it can recognize multiple antigenic epitopes. In the present disclosure, at least one of the plurality of antigenic epitopes is derived from the first molecule.

According to an embodiment of the present disclosure, the monoclonal antibody includes at least one of a Fab antibody, a F(ab')₂ fragment, a Fv antibody, a single chain antibody, a single domain antibody, and a minimal recognition unit.

As used herein, the terms "single domain antibody", "nanoantibody", and "VHH antibody" are used interchangeably, which were originally described as antigen-binding immunoglobulin (variable) domains of "heavy chain antibodies" (i.e., "antibodies lacking light chains") (Hamers-Casterman C, Atarhouch T, Muyldermans S, Robinson G, Hamers C, Songa EB, Bendahman N, Hamers R.: "Naturally occurring antibodies devoid of light chains"; Nature 363, 446-448 (1993)), including a heavy chain variable region (VH) and conventional CH2 and CH3 regions, which specifically bind to antigen proteins (e.g., D-dimers) through the heavy chain variable region.

As used herein, the term "Fab antibody" or "Fab fragment" generally refers to an antibody or fragment containing only the Fab molecule, which is composed of the VH and CH1 of the heavy chain and a complete light chain, with the light chain and the heavy chain linked by a disulfide bond.

As used herein, the term "F(ab')₂ antibody" or "F(ab')₂ fragment" has two antigen-binding F(ab') portions linked together by a disulfide bond.

As used herein, the term "Fv antibody" or "Fv fragment" generally refers to an antibody or fragment consisting only of a light chain variable region (VL) and a heavy chain variable region (VH) linked by non-covalent bonds, and is the smallest functional fragment of an antibody that retains a complete antigen binding site.

As used herein, the terms "single chain antibody" and "scFv fragment" refer to an antibody or fragment formed by linking the heavy chain variable region and the light chain variable region of an antibody via a short peptide.

As used herein, the terms "minimal recognition unit" and "MRU" both refer to an antibody or fragment consisting of only one CDR, which has a very small molecular weight, accounting for only about 1% of the complete antibody.

According to an embodiment of the present disclosure, the intracellular region includes at least one of a transcription activator protein, a transcription repressor protein, a transcription factor, a site-specific nuclease, a recombinase, an activating immune receptor intracellular domain, and an inhibitory immune receptor intracellular domain.

According to an embodiment of the present disclosure, the intracellular region includes at least one of GaL4 VP64, GaL4 VP16, tetR VP64, ZFHD1 VP64, Gal4 KRAB, HAP1 VP16, LexA VP64, Cas9, and Cas13.

According to an embodiment of the present disclosure, the Gal4 VP64 has an amino acid sequence as set forth in SEQ ID NO: 2.

MKLLSSIEQACDICRLKKLKCSKEKPKCAKCLKNNWECRYSPKTKRSPLTR
AHLTEVESRLERLEQLFLLIFPREDLDMILKMDSLQDIKALLTGLFVQDNVNKDAVTD
RLASVETDMPLTLRQHRISATSSSEESSNKGQRQLTVSAAAGGSGGSGGSDALDDFDL
DMLGSDALDDFDLDMLGSDALDDFDLDMLGSDALDDFDLDMLGS (SEQ ID NO: 2).

According to an embodiment of the present disclosure, the transmembrane region and the intracellular region have an amino acid sequence as set forth in SEQ ID NO: 3.

VVSPCASRPCYNGGTCQFSPEEPFFQCFCPTNFNGLFCHILDYGFIGGLGKN
ITPPDNEEICENEQCAELADNKICNANCNNHACGWDGGDCSLNFNDPWKNCTQSLQ

CWKYFNDGKCDSQCNNSGCLYDGFDCQKVEVQCNPLYDQYCRDHFQDGHCDQGC
NNAECEWDGLDCDNMPENLAEGTLLIVVLMPPEKLKNNSVNFLRELSRVLHTNVVF
KKDSKGEYKIYPYYGNEEELKKHHIKKRSAASWSDAPTAIFSTMKESVLPGRRRREL
DQMEVRGSIVYLEIDNRQCYKSSSQCFTSATDVAAFLGALATHGNLNIPYKIEAVKSEI
VETAKPPPPLYAMFSMLVIPLLIIFVIMVVIVNKKRRREHGQLWFPMKLLSSIEQACDIC
RLKKLKCSKEKPKCAKCLKNNWECRYSPKTKRSPLTRAHLTEVESRLERLEQLFLLIF
PREDLDMILKMDSLQDIKALLTGLFVQDNVNKDAVTDRLASVETDMPLTLRQHRISA
TSSSEESSNKGQRQLTVSAAAGGSGGSGGSDALDDFDLDMLGSDALDDFDLDMLGS
DALDDFDLDMLGSDALDDFDLDMLGS (SEQ ID NO: 3).

According to an embodiment of the present disclosure, the chimeric polypeptide has an amino acid sequence as set forth in SEQ ID NO: 4.

MALPVTALLLPLALLLHAARPEQKLISEEDLMADVQLVESGGGLVQAGGS
LRLSCAASGRTISMAAMSWFRQAPGKEREFVAGISRSAGSAVHADSVKGRFTISRDNT
KNTLYLQMNSLKAEDTAVYYCAVRTSGFFGSIPRTGTAFDYWGQGTQVTVSVVSPCA
SRPCYNGGTCQFSPEEPFFQCFCPTNFNGLFCHILDYGFIGGLGKNITPPDNEEICENEQ
CAELADNKICNANCNNHACGWDGGDCSLNFNDPWKNCTQSLQCWKYFNDGKCDS
QCNNSGCLYDGFDCQKVEVQCNPLYDQYCRDHFQDGHCDQGCNNAECEWDGLDC
DNMPENLAEGTLLIVVLMPPEKLKNNSVNFLRELSRVLHTNVVFKKDSKGEYKIYPY
YGNEEELKKHHIKKRSAASWSDAPTAIFSTMKESVLPGRRRRELDQMEVRGSIVYLEI
DNRQCYKSSSQCFTSATDVAAFLGALATHGNLNIPYKIEAVKSEIVETAKPPPPLYAMF
SMLVIPLLIIFVIMVVIVNKKRRREHGQLWFPMKLLSSIEQACDICRLKKLKCSKEKPK
CAKCLKNNWECRYSPKTKRSPLTRAHLTEVESRLERLEQLFLLIFPREDLDMILKMDS
LQDIKALLTGLFVQDNVNKDAVTDRLASVETDMPLTLRQHRISATSSSEESSNKGQRQ
LTVSAAAGGSGGSGGSDALDDFDLDMLGSDALDDFDLDMLGSDALDDFDLDMLGS
DALDDFDLDMLGS (SEQ ID NO:4).

### Nucleic acid molecule, vector, recombinant cell, and pharmaceutical composition

In yet another aspect of the present disclosure, the present disclosure provides a first nucleic acid molecule. According to an embodiment of the present disclosure, the first nucleic acid molecule encodes the above-mentioned chimeric polypeptide. The first nucleic acid molecule according to the embodiment of the present disclosure can encode the above-mentioned chimeric polypeptide.

According to an embodiment of the present disclosure, the first nucleic acid molecule is DNA.

It should be noted that, with respect to the first nucleic acid molecule referred to herein, those skilled in the art will understand that it actually includes either or both complementary double strands. For convenience, as used herein, although in most cases only one strand is indicated, another strand complementary thereto is actually disclosed. In addition, the molecule sequences in the present disclosure include DNA forms or RNA forms, one of which is disclosed, meaning that the other is also disclosed.

In yet another aspect of the present disclosure, the present disclosure provides a first expression vector. According to an embodiment of the present disclosure, the first expression vector carries the above-mentioned first nucleic acid molecule. When the above-mentioned first nucleic acid molecule is linked to a vector, the first nucleic acid molecule can be directly or indirectly linked to the control elements on the vector, as long as these control elements can control the translation and expression of the first nucleic acid molecule. Of course, these control elements may come directly from the vector itself, or they may be exogenous, that is, not from the vector itself. Of course, it is sufficient that the first nucleic acid molecule is operably linked to the control element.

As used herein, "operably linked" means linking the exogenous gene to the vector to enable the control elements on the vector, such as transcription control sequences and translation control sequences, etc., play their intended function of regulating the transcription and translation of the exogenous gene. Commonly used vectors include plasmids, phages, and the like. After the vectors according to some specific embodiments of the present disclosure are introduced into a suitable receiver cell (also called a receptor cell or a host cell), the expression of the above-mentioned chimeric polypeptide can be effectively achieved under the mediation of the regulatory system.

According to an embodiment of the present disclosure, the first expression vector is a eukaryotic expression vector, a prokaryotic expression vector, a virus, or a bacteriophage.

According to an embodiment of the present disclosure, the first expression vector is a plasmid expression vector.

In yet another aspect of the present disclosure, the present disclosure provides a recombinant cell. According to an embodiment of the present disclosure, the recombinant cell carries the above-mentioned first nucleic acid molecule or the above-mentioned first expression vector; or expresses the above-mentioned chimeric polypeptide. The above-mentioned chimeric polypeptide can be effectively expressed in the recombinant cell under suitable conditions.

It should be noted that "suitable conditions" in the description of the present disclosure refer to the conditions suitable for the expression of the above-mentioned chimeric polypeptide. Those skilled in the art can readily appreciate that the conditions suitable for the expression of the above-mentioned chimeric polypeptide include, but are not limited to, a suitable transformation or transfection method, a suitable transformation or transfection condition, a healthy cell state, a suitable cell density, a suitable cell culture environment, and a suitable cell culture time. The "suitable conditions" are not particularly limited, and those skilled in the art can optimize the most suitable conditions for the expression of the chimeric polypeptide according to the specific circumstances of the laboratory.

According to an embodiment of the present disclosure, the recombinant cell is obtained by introducing the above-mentioned first expression vector into a host cell.

According to an embodiment of the present disclosure, the host cell includes at least one of an immune cell, a neuron, a progenitor cell or a precursor cell, an epithelial cell, an endothelial cell, and a stem cell.

According to an embodiment of the present disclosure, the host cell includes at least one of a T cell, a B cell, a monocyte, a NK cell, a dendritic cell, a macrophage, a regulatory T cell, a helper T cell, a cytotoxic T cell, a NKT cell, and a γδ T cell.

According to an embodiment of the present disclosure, the recombinant cell further includes: a second nucleic acid molecule encoding a chimeric antigen receptor, a NK cell receptor, or a T cell receptor, or a second expression vector carrying the second nucleic acid molecule; or the recombinant cell expresses the chimeric antigen receptor, the T cell receptor, or the NK cell receptor. The inventors through experiments found that by combining the chimeric polypeptide of the present disclosure with a chimeric antigen receptor, a NK cell receptor, or a T cell receptor to jointly modify the immune cells (such as Jurkat T cells and NK cells), the immune cells combined with the chimeric polypeptide +CAR/TCR can recognize the corresponding ligands and efficiently activate the immune cells (such as Jurkat T cells and NK cells) to exercise the function of killing targets (such as tumor cells, etc.)

As used herein, the term "chimeric antigen receptor (CAR)" is a fusion protein including an extracellular domain capable of binding to an antigen, a transmembrane domain derived from a different polypeptide than the extracellular domain, and at least one intracellular domain. "Chimeric antigen receptor (CAR)" is also known as "chimeric receptor", "T-body", or "chimeric immune receptor (CIR)". The "extracellular domain capable of binding to an antigen" refers to any oligopeptide or polypeptide capable of binding to a certain antigen. "Intracellular domain" refers to any oligopeptide or polypeptide known to function as a domain that transmits signals to activate or inhibit biological processes within a cell.

As used herein, the term "T cell receptor (TCR)" refers to a molecule found on the surface of T cells that is responsible for recognizing antigens that are bound to MHC molecules. Naturally occurring TCR heterodimers are composed of alpha (α) and beta (β) chains in approximately 95% of T cells, while approximately 5% of T cells have TCRs composed of gamma (γ) and delta (δ) chains. During antigen processing, the antigen is degraded inside the cell and then carried to the cell surface by major histocompatibility complex (MHC) molecules. T cells can recognize this antigen peptide-MHC complex on the surface of antigen presenting cells. The binding of TCR to the antigen peptide-MHC complex leads to the activation of T lymphocytes, and TCR is expressed on T lymphocytes through a series of biochemical reactions mediated by related enzymes, co-receptors, and specialized auxiliary molecules. The MHC molecule may be a class I or class II MHC molecule. The complex can be present in an antigen presenting cell, such as a dendritic cell, a B cell, or any other cell (e.g., a K562 cell). The human leukocyte antigen system (HLA) is the name of the gene complex encoding the human major histocompatibility complex (MHC), and includes HLA class I antigens (A, B, and C) and HLA class II antigens (DP, DQ, and DR). HLA alleles A, B, and C present peptides that are primarily derived from intracellular proteins, e.g., proteins expressed within cells.

As used herein, the term "NK cell receptor" refers to a molecule that can be found on the surface of NK cells and can be divided into inhibitory receptors and activating receptors based on the different functions it mediates. Among them, the inhibitory receptors are used to transduce killing inhibition signals, and inhibit their own killing function after recognizing specific molecules expressed on the surface of normal cells. There are many types of inhibitory receptors, including but not limited to CD161, CLRG1, PD1, TIM3, LAG3, CD96, and TIGIT. The activating receptors are used to transduce activation signals into the cells, and exert a killing effect after recognizing the corresponding ligands on the surface of target cells. The activating receptors include, but are not limited to, NKp30, NKp44, NKp46, and CD16.

According to an embodiment of the present disclosure, an intracellular region includes a transcription activator protein. 5'-end of the second nucleic acid molecule encoding the chimeric antigen receptor, the NK cell receptor, or the T cell receptor is linked to an inducible expression nucleic acid sequence for binding to the transcription activator protein.

Exemplarily, the intracellular region is GaL4 VP64, and the inducible expression nucleic acid sequence is a UAS-minimal-CMV sequence.

The UAS-minimal-CMV sequence has a nucleic acid sequence as set forth in SEQ ID NO: 14.

GGAGCACTGTCCTCCGAACGTCGGAGCACTGTCCTCCGAACGTCGGAG
CACTGTCCTCCGAACGTCGGAGCACTGTCCTCCGAACGGAGCATGTCCTCCGAAC
GTCGGAGCACTGTCCTCCGAACGACTAGTTAGGCGTGTACGGTGGGAGGCCTATAT
AAGCAGAGCTCGTTTAGTGAACCGTCAGATCGCCTGGAGACGCCATCCACGCTGT
TTTGACCTCCATAGAAGACACCGGGACCGATCCAGC (SEQ ID NO: 14).

In an optional embodiment of the present disclosure, the chimeric antigen receptor, the NK cell receptor, or the T cell receptor has an activity of binding to a second molecule. For example, the chimeric antigen receptor binds to the second molecule mainly through its extracellular region.

According to an embodiment of the present disclosure, the second molecule includes at least one of a tumor antigen, a virus, a bacterium, an endotoxin, an antibody, a cell receptor, and a ligand of a cell receptor.

In an optional embodiment of the present disclosure, the second molecule includes at least one of GFP, eGFP, CD19, ALPPL2, BCMA, SIRPα, CD1a, CD1b, CD1c, CD1d, CD1e, CD2, CD3d, CD3e, CD3g, CD4, CD5, CD7, CD8a, CD8b, CD20, CD21, CD22, CD23, CD25, CD27, CD28, CD30, CD33, CD34, CD38, CD40, CD44, CD44v6, CD45, CD48, CD51, CD52, CD56, CD59, CD66, CD70, CD71, CD72, CD73, CD74, CD79A, CD79B, CD80, CD86, CD94, CD95, CD133, CD134, CD140, CD152, CD154, CD158, CD178, CD181, CD182, CD183, CD200, CD210, CD221, CD246, CD252, CD253, CD261, CD262, CD273, CD274, CD276, CD279, CD295, CD339, CD340, EGFR, HER2, FGFR2, AFP, CA125, MSLN, GPC3, CEA, CLDN18.2, EpCAM, PSCA, GD2, IL-13, IL-13RA2, ROR1, MUC 1, PSMA, MAGEA1, 4-1BB, 5T4, BAFF, CA242, CA-IX, MET, CCR4, CNTO888, FAP, MORAb-009, VEGF-A, VEGFR-1, and VEGFR-2.

In yet another aspect of the present disclosure, the present disclosure provides a pharmaceutical composition. According to an embodiment of the present disclosure, the pharmaceutical composition includes the above-mentioned chimeric polypeptide, the above-mentioned first nucleic acid molecule, the above-mentioned first expression vector, or the above-mentioned recombinant cell. As can be seen from the foregoing, the cell expressing the above-mentioned chimeric polypeptide can recognize the first molecule, and after binding to the first molecule, the cell can realize many different signal output types, such as activation and inhibition of specific gene expression. Alternatively, the immune cells expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. Therefore, the pharmaceutical composition containing the above-mentioned chimeric polypeptide, first nucleic acid molecule, first expression vector, or recombinant cell can target the first molecule for preventing and/or treating related diseases, such as cancer.

According to an embodiment of the present disclosure, the pharmaceutical composition further includes a pharmaceutically acceptable excipient.

### Use

In another aspect of the present disclosure, the present disclosure provides a use of the above-mentioned chimeric polypeptide, the above-mentioned first nucleic acid molecule, the above-mentioned first expression vector, the above-mentioned recombinant cell, or the above-mentioned pharmaceutical composition in the preparation of a medicament for preventing and/or treating a disease. As can be seen from the foregoing, the cell expressing the above-mentioned chimeric polypeptide can recognize the first molecule, and after binding to the first molecule, the cell can realize many different signal output types, such as activation and inhibition of specific gene expression. Alternatively, the immune cells expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. In addition, the pharmaceutical composition includes the above-mentioned chimeric polypeptide, the first nucleic acid molecule, the first expression vector, or the recombinant cell. Therefore, the medicament containing the above-mentioned chimeric polypeptide, first nucleic acid molecule, first expression vector, recombinant cell, or pharmaceutical composition can target the first molecule for preventing and/or treating related diseases, such as cancer.

In another aspect of the present disclosure, the present disclosure provides the above-mentioned chimeric polypeptide, the above-mentioned first nucleic acid molecule, the above-mentioned first expression vector, the above-mentioned recombinant cell, or the above-mentioned pharmaceutical composition for use in the prevention and/or treatment of a disease. As can be seen from the foregoing, the cell expressing the above-mentioned chimeric polypeptide can recognize the first molecule, and after binding to the first molecule, the cell can realize many different signal output types, such as activation and inhibition of specific gene expression. Alternatively, the immune cell expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. In addition, the pharmaceutical composition includes the above-mentioned chimeric polypeptide, the first nucleic acid molecule, the first expression vector, or the recombinant cell. Therefore, the medicament containing the above-mentioned chimeric polypeptide, first nucleic acid molecule, first expression vector, recombinant cell, or pharmaceutical composition can target the first molecule for preventing and/or treating related diseases, such as cancer.

According to an embodiment of the present disclosure, the use of the above-mentioned two aspects can further include at least one of the following technical features.

According to an embodiment of the present disclosure, the disease includes cancer or tumor and immune-related disease.

According to an embodiment of the present disclosure, the disease includes cancer or tumor, autoimmune disease, inflammation, and related diseases caused by cellular senescence.

As used herein, the term "cancer" or "tumor" may refer to any unregulated cell growth. Exemplarily, it may be non-small cell lung cancer, papillary thyroid cancer, glioblastoma multiforme, colon cancer, rectal cancer, lung cancer, head and neck cancer, renal cancer, bladder cancer, breast cancer, ovarian cancer, liver cancer, bile duct cancer or sarcoma, acute myeloid leukemia, large-cell neuroendocrine carcinoma, neuroblastoma, prostate cancer, neuroblastoma, pancreatic cancer, melanoma, head and neck squamous cell carcinoma, cervical cancer, skin cancer, glioma, esophageal cancer, oral squamous cell carcinoma, or gastric cancer, etc.

### Method

In yet another aspect of the present disclosure, the present disclosure provides a method for activating an immune cell. According to an embodiment of the present disclosure, the method includes: performing a first contact on the immune cell and a first molecule, wherein the immune cell expresses the above-mentioned chimeric polypeptide. Therefore, the immune cell expressing the above-mentioned chimeric polypeptide can recognize the first molecule, and the immune cell can be activated after the chimeric polypeptide binding to the first molecule. The method is particularly suitable for scientific research, and activated immune cells can be obtained by culturing the immune cell in vitro.

According to an embodiment of the present disclosure, the intracellular region of the chimeric polypeptide includes a transcription activator protein; the immune cell expresses a chimeric antigen receptor, a NK cell receptor, or a T cell receptor. The chimeric antigen receptor, the NK cell receptor, or the T cell receptor includes an antibody or a functional fragment thereof that binds to a predetermined antigen; and 5'-end of a second nucleic acid molecule encoding the chimeric antigen receptor, the NK cell receptor, or the T cell receptor is linked to an inducible expression nucleic acid sequence for binding to the transcription activator protein. The method further includes, subsequent to the first contact: releasing, by the immune cell, the transcription activator protein, and expressing, by the immune cell, the chimeric antigen receptor, the NK cell receptor, or the T cell receptor; and activating the immune cell by binding the chimeric antigen receptor, the NK cell receptor, or the T cell receptor of the immune cell to the predetermined antigen.

According to an embodiment of the present disclosure, the inducible expression nucleic acid sequence has a nucleic acid sequence as set forth in SEQ ID NO: 14.

According to an embodiment of the present disclosure, the first molecule is a tumor antigen.

In an optional embodiment of the present disclosure, the first molecule includes at least one of GFP, eGFP, CD19, ALPPL2, BCMA, SIRPα, CD1a, CD1b, CD1c, CD1d, CD1e, CD2, CD3d, CD3e, CD3g, CD4, CD5, CD7, CD8a, CD8b, CD20, CD21, CD22, CD23, CD25, CD27, CD28, CD30, CD33, CD34, CD38, CD40, CD44, CD44v6, CD45, CD48, CD51, CD52, CD56, CD59, CD66, CD70, CD71, CD72, CD73, CD74, CD79A, CD79B, CD80, CD86, CD94, CD95, CD133, CD134, CD140, CD152, CD154, CD158, CD178, CD181, CD182, CD183, CD200, CD210, CD221, CD246, CD252, CD253, CD261, CD262, CD273, CD274, CD276, CD279, CD295, CD339, CD340, EGFR, HER2, FGFR2, AFP, CA125, MSLN, GPC3, CEA, CLDN18.2, EpCAM, PSCA, GD2, IL-13, IL-13RA2, ROR1, MUC 1, PSMA, MAGEA1, 4-1BB, 5T4, BAFF, CA242, CA-IX, MET, CCR4, CNTO888, FAP, MORAb-009, VEGF-A, VEGFR-1, and VEGFR-2.

According to an embodiment of the present disclosure, the immune cell includes at least one of a T cell, a B cell, a monocyte, a NK cell, a dendritic cell, a macrophage, a regulatory T cell, a helper T cell, a cytotoxic T cell, a NKT cell, and a γδ T cell.

In yet another aspect of the present disclosure, the present disclosure provides a method for tracking a first cell-second cell contact. According to an embodiment of the present disclosure, the method includes: performing a second contact on the first cell and the second cell, wherein the first cell expresses the above-mentioned chimeric polypeptide and a reporter gene protein, the intracellular region of the chimeric polypeptide being a transcription activator protein, and 5'-end of a third nucleic acid molecule encoding the reporter gene protein being linked to an inducible expression nucleic acid sequence for binding to the transcription activator protein, and wherein the second cell expresses the first molecule; and determining a contact situation between the first cell and the second cell based on a detection result of the reporter gene protein in the first cell. Therefore, the above-mentioned method can be used to determine whether the first cell and the second cell are in contact based on the detection result of the reporter gene protein. The method is particularly suitable for scientific research and can be used to determine whether two cells are in contact in vitro.

According to an embodiment of the present disclosure, the inducible expression nucleic acid sequence has a nucleic acid sequence as set forth in SEQ ID NO: 14.

According to an embodiment of the present disclosure, the first molecule is on the surface of the second cell, is immobilized on an insoluble substrate, exists in an extracellular matrix, exists in an artificial matrix, or is soluble.

According to an embodiment of the present disclosure, the first molecule is a tumor antigen.

In an optional embodiment of the present disclosure, the first molecule includes at least one of GFP, eGFP, CD19, ALPPL2, BCMA, SIRPα, CD1a, CD1b, CD1c, CD1d, CD1e, CD2, CD3d, CD3e, CD3g, CD4, CD5, CD7, CD8a, CD8b, CD20, CD21, CD22, CD23, CD25, CD27, CD28, CD30, CD33, CD34, CD38, CD40, CD44, CD44v6, CD45, CD48, CD51, CD52, CD56, CD59, CD66, CD70, CD71, CD72, CD73, CD74, CD79A, CD79B, CD80, CD86, CD94, CD95, CD133, CD134, CD140, CD152, CD154, CD158, CD178, CD181, CD182, CD183, CD200, CD210, CD221, CD246, CD252, CD253, CD261, CD262, CD273, CD274, CD276, CD279, CD295, CD339, CD340, EGFR, HER2, FGFR2, AFP, CA125, MSLN, GPC3, CEA, CLDN18.2, EpCAM, PSCA, GD2, IL-13, IL-13RA2, ROR1, MUC 1, PSMA, MAGEA1, 4-1BB, 5T4, BAFF, CA242, CA-IX, MET, CCR4, CNTO888, FAP, MORAb-009, VEGF-A, VEGFR-1, and VEGFR-2.

According to an embodiment of the present disclosure, the first cell or the second cell includes at least one of an immune cell, a neuron, a progenitor cell or a precursor cell, an epithelial cell, an endothelial cell, and a stem cell.

According to an embodiment of the present disclosure, the first cell includes at least one of a T cell, a B cell, a monocyte, a NK cell, a dendritic cell, a macrophage, a regulatory T cell, a helper T cell, a cytotoxic T cell, a NKT cell, and a γδ T cell.

In yet another aspect of the present disclosure, the present disclosure provides a method for regulating cell activity. According to an embodiment of the present disclosure, the method includes: performing a third contact on the cell and a first molecule, wherein the cell expresses the above-mentioned chimeric polypeptide.

According to an embodiment of the present disclosure, the cell includes at least one of an immune cell, a neuron, a progenitor cell or a precursor cell, an epithelial cell, an endothelial cell, and a stem cell.

According to an embodiment of the present disclosure, the cell includes at least one of a T cell, a B cell, a monocyte, a NK cell, a dendritic cell, a macrophage, a regulatory T cell, a helper T cell, a cytotoxic T cell, a NKT cell, and a γδ T cell.

In yet another aspect of the present disclosure, the present disclosure provides a method for promoting the expression of genes or proteins in cells. According to an embodiment of the present disclosure, the method includes: performing a fourth contact on the cell and a first molecule, wherein the cell expresses the above-mentioned chimeric polypeptide, and the cell carries the gene or expresses the protein; wherein the gene includes a nucleic acid molecule encoding at least one of a chimeric antigen receptor, a second chimeric polypeptide, a translation regulator, a cytokine, a hormone, a chemokine, an antibody, and a protein located in the intracellular region, or the protein includes at least one of a chimeric antigen receptor, a second chimeric polypeptide, a translation regulator, a cytokine, a hormone, a chemokine, an antibody, and a protein located in the intracellular region.

According to an embodiment of the present disclosure, the intracellular region of the chimeric polypeptide includes a transcription activator protein, and the chimeric antigen receptor, the second chimeric polypeptide, the translation regulator, the cytokine, the hormone, the chemokine, or the antibody includes a second binding protein or a fragment thereof of the transcription activator protein.

According to an embodiment of the present disclosure, the cell includes at least one of an immune cell, a neuron, a progenitor cell or a precursor cell, an epithelial cell, an endothelial cell, and a stem cell.

According to an embodiment of the present disclosure, the cell includes at least one of a T cell, a B cell, a monocyte, a NK cell, a dendritic cell, a macrophage, a regulatory T cell, a helper T cell, a cytotoxic T cell, a NKT cell, and a γδ T cell.

In yet another aspect of the present disclosure, the present disclosure provides a method for treating or preventing a disease. According to an embodiment of the present disclosure, the method includes: administering a pharmaceutically acceptable dose of the above-mentioned recombinant cell or the above-mentioned pharmaceutical composition to a subject. As can be seen from the foregoing, the cell expressing the above-mentioned chimeric polypeptide can recognize the first molecule, and after binding to the first molecule, the cell can realize many different signal output types, such as activation and inhibition of specific gene expression. Alternatively, the immune cell expressing the chimeric polypeptide can secrete factors with therapeutic effects (such as single chain antibodies, cytokines, etc.) for anti-tumor after contacting the first molecule. Moreover, the first nucleic acid molecule and the first expression vector can express the chimeric polypeptide in cells (such as immune cells), and the chimeric polypeptide can be expressed on the above-mentioned recombinant cells. In addition, the pharmaceutical composition includes the above-mentioned chimeric polypeptide, the first nucleic acid molecule, the first expression vector, or the recombinant cell. Therefore, the medicament containing the recombinant cell or pharmaceutical composition which includes the above-mentioned chimeric polypeptide can target the first molecule for preventing and/or treating related diseases, such as cancer.

The effective amount of the recombinant protein or pharmaceutical composition of the present disclosure may vary depending on the mode of administration and the severity of the disease to be treated. The selection of a preferred effective amount may be determined by those skilled in the art based on various factors (e.g. through clinical trials). The factors include, but are not limited to: pharmacokinetic parameters of the active ingredient such as bioavailability, metabolism, half-life, etc.; the severity of the disease to be treated, the weight of the patient, the immune status of the patient, the route of administration, etc. For example, several separate doses may be administered daily due to the urgent demands of the treatment condition, or the dose may be proportionally reduced.

The recombinant protein or pharmaceutical composition of the present disclosure can be incorporated into drugs suitable for parenteral administration (e.g., intravenous, subcutaneous, intraperitoneal, and intramuscular). These medicaments can be prepared in a variety of forms. For example, liquid, semi-solid and solid dosage forms, etc., including but not limited to liquid solution (e.g., injection solution and infusion solution) or lyophilized powders. The drug is typically in the form of an injectable solution or an infusible solution. The above-mentioned recombinant protein or pharmaceutical composition can be administered by intravenous infusion or injection or intramuscular or subcutaneous injection.

According to an embodiment of the present disclosure, the administration route of the method is subcutaneous injection or intravenous injection.

According to an embodiment of the present disclosure, the disease includes cancer or tumor, and immune-related disease. According to an embodiment of the present disclosure, the disease includes cancer or tumor, autoimmune disease, inflammation, and related diseases caused by cellular senescence.

According to an embodiment of the present disclosure, the tumor or cancer includes but is not limited to non-small cell lung cancer, papillary thyroid cancer, glioblastoma multiforme, colon cancer, rectal cancer, lung cancer, head and neck cancer, renal cancer, bladder cancer, breast cancer, ovarian cancer, liver cancer, bile duct cancer or sarcoma, acute myeloid leukemia, large-cell neuroendocrine carcinoma, neuroblastoma, prostate cancer, neuroblastoma, pancreatic cancer, melanoma, head and neck squamous cell carcinoma, cervical cancer, skin cancer, glioma, esophageal cancer, oral squamous cell carcinoma, or gastric cancer, etc.

The solution of the present disclosure will be illustrated below with reference to examples. Those skilled in the art will appreciate that the following examples are only used to illustrate the present disclosure and should not be construed as limiting the scope of the present disclosure. Where specific techniques or conditions are not specified in the examples, they are performed according to techniques or conditions described in the literature in the art or according to the product description. The reagents or instruments used without indicating the manufacturer are conventional products that can be obtained commercially.

### Example 1: Design of SynNotch synthetic receptor and construction of its expression plasmid vector

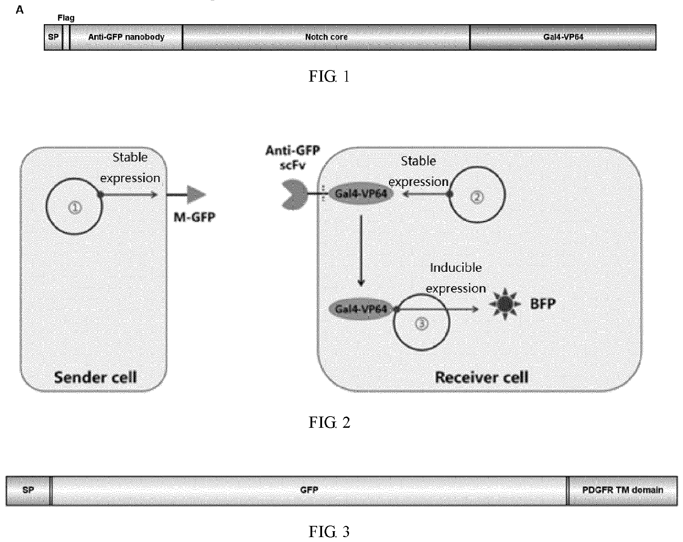

1. The structure of the SynNotch synthetic receptor (abbreviated as SynNotch receptor) includes an extracellular domain (also known as extracellular region), a Notch transmembrane domain (also known as transmembrane region of a Notch receptor protein, abbreviated as transmembrane region), and an intracellular domain (also known as intracellular region), wherein the extracellular domain can be replaced by a single chain antibody or a nano antibody, and the intracellular domain can be replaced by a transcription activation domain or transcription repression domain. The present disclosure used an anti-GFP single chain antibody as the extracellular domain, GAL4 VP64 as the intracellular domain, and a Notch transmembrane domain of a mouse or *Xenopus tropicalisthat* can be recognized and cleaved by a protease as the Notch transmembrane domain to obtain a modified SynNotch receptor. The structure of the SynNotch receptor designed and modified by the present disclosure was shown in FIG 1. FIG 1 showed the structure of a SynNotch receptor including the Notch transmembrane domain (Notch core) of mouse or *Xenopus tropicalis.*
2. Construction of the expression plasmid vector of the above-mentioned SynNotch synthetic receptor. The specific steps were as follows.
   (1) The SynNotch synthetic receptor containing the Notch transmembrane domain of *Xenopus tropicalis* (referred to as SynNotch synthetic receptor 1) has an amino acid sequence as set forth in SEQ ID NO: 4 and a nucleotide sequence as set forth in SEQ ID NO: 5, and the SynNotch synthetic receptor containing the Notch transmembrane domain of mouse (referred to as SynNotch synthetic receptor 2) has an amino acid sequence as set forth in SEQ ID NO: 6 and a nucleotide sequence as set forth in SEQ ID NO: 7, and then the nucleotide sequence as set forth in SEQ ID NO: 5 and the nucleotide sequence as set forth in SEQ ID NO: 7 were synthesized into a Puc57 vector by GENEWIZ, Inc., respectively.
   (2) The pCDH-EV vector was double-digested with nucleic acid endonucleases EocR1 and BamH1, and the double-digested vector was recovered by gel purification after electrophoresis using a 1% agarose gel for 20 minutes;
   (3) The nucleotide sequence as set forth in SEQ ID NO: 5 and the nucleotide sequence as set forth in SEQ ID NO: 7 were obtained from the two pUC57 vectors in step (1) by PCR using primer pairs, and then the nucleotide sequence as set forth in SEQ ID NO: 5 and the nucleotide sequence as set forth in SEQ ID NO: 7 were homologously recombined with the double-digested vector obtained in step (2) and the nucleotide fragments of the SynNotch synthetic receptor, respectively, to obtain two expression plasmid vectors pCDH-antiGFP-XENTR-SynNotch-Gal4-VP64 and pCDH-antiGFP-MOUSE-SynNotch-Gal4-VP64.
   (4) The two expression plasmid vectors constructed above (PCDH-antiGFP-XENTR-SynNotch-Gal4VP64 and pCDH-antiGFP-MOUSE-SynNotch-Gal4-VP64) were sequenced and verified, and the sequencing results confirmed that the constructions were successful.

ATGGCATTGCCCGTGACCGCCCTGCTGCTGCCACTGGCCTTGTTGCTCC
ACGCCGCGCGGCCAGAACAGAAGCTGATCAGCGAGGAGGATCTGATGGCTGATGT
GCAACTCGTAGAGTCCGGTGGCGGACTCGTCCAGGCGGGAGGCTCCCTGCGCCTG
AGTTGCGCTGCGTCCGGAAGAACTATTAGCATGGCCGCCATGAGTTGGTTCAGACA
GGCGCCTGGAAAGGAGAGGGAGTTCGTGGCAGGCATAAGTCGCTCCGCAGGGTC
AGCCGTCCATGCCGACTCCGTAAAGGGACGCTTTACCATCTCCAGAGATAACACAA
AAAACACGCTCTATCTCCAAATGAATTCTTTGAAGGCCGAGGACACAGCCGTGTAC
TACTGCGCCGTTAGGACCAGCGGATTTTTTGGAAGCATTCCTAGGACAGGGACAG
CCTTTGATTACTGGGGCCAGGGAACGCAGGTTACCGTTTCCGTCGTCAGCCCGTGC
GCGTCCCGCCCTTGTTACAACGGAGGGACCTGCCAATTCTCCCCCGAGGAACCTTT
CTTCCAGTGCTTCTGCCCCACGAACTTCAACGGCCTCTTCTGCCACATCTTGGATT
ACGGCTTCATCGGAGGCCTGGGCAAGAACATCACCCCTCCCGACAATGAGGAAAT
CTGCGAGAATGAGCAGTGCGCCGAGCTGGCCGACAACAAGATCTGCAACGCCAA
CTGCAACAACCACGCCTGCGGGTGGGACGGCGGCGACTGCTCTCTCAACTTCAAC
GACCCCTGGAAGAACTGCACCCAGTCGCTGCAGTGCTGGAAATACTTCAACGACG
GCAAATGCGACTCGCAGTGCAACAACTCCGGCTGCCTGTACGACGGCTTCGACTG

CCAGAAAGTGGAGGTTCAGTGCAACCCTTTGTACGACCAGTATTGCAGGGATCAC
TTTCAAGACGGCCATTGCGACCAAGGCTGTAACAACGCAGAGTGCGAATGGGACG
GCCTGGACTGTGACAACATGCCGGAGAACCTGGCCGAAGGCACCCTGTTGATAGT
CGTCCTGATGCCCCCCGAAAAGCTGAAGAACAACTCCGTCAACTTCCTCCGAGAG
CTGAGCCGAGTGCTCCACACCAACGTGGTGTTCAAGAAAGACAGCAAAGGCGAA
TACAAGATCTACCCTTACTATGGCAACGAGGAGGAGCTGAAGAAACATCACATCA
AGAAGAGGTCGGCGGCTTCCTGGTCGGATGCTCCTACCGCCATCTTCAGTACCATG
AAGGAGTCCGTCTTACCAGGAAGGCGCCGTAGGGAGTTGGACCAAATGGAAGTC
AGGGGGTCAATCGTATACTTAGAAATAGACAACCGCCAGTGCTACAAATCCTCCTC
CCAGTGCTTCACCAGCGCCACCGACGTGGCCGCCTTCCTCGGGGCTTTGGCCACC
CATGGCAATCTGAACATCCCCTATAAGATCGAGGCTGTGAAAAGTGAGATAGTCGA
GACCGCCAAGCCGCCGCCGCCGCTTTATGCCATGTTTTCCATGTTGGTCATCCCGTT
GCTGATCATCTTCGTCATCATGGTGGTCATCGTGAATAAGAAGCGGCGCCGCGAAC
ACGGGCAGCTGTGGTTCCCCATGAAGCTGCTGAGCAGCATCGAGCAGGCCTGTGA
CATCTGCCGGCTGAAGAAACTGAAGTGCAGCAAAGAAAAGCCCAAGTGCGCCAA
GTGCCTGAAGAACAACTGGGAGTGCCGGTACAGCCCCAAGACCAAGAGAAGCCC
CCTGACCAGAGCCCACCTGACCGAGGTGGAAAGCCGGCTGGAAAGACTGGAACA
GCTGTTTCTGCTGATCTTCCCACGCGAGGACCTGGACATGATCCTGAAGATGGACA
GCCTGCAGGACATCAAGGCCCTGCTGACCGGCCTGTTCGTGCAGGACAACGTGAA
CAAGGACGCCGTGACCGACAGACTGGCCAGCGTGGAAACCGACATGCCCCTGAC
CCTGCGGCAGCACAGAATCAGCGCCACCAGCAGCAGCGAGGAAAGCAGCAACAA
GGGCCAGCGGCAGCTGACAGTGTCTGCTGCTGCAGGCGGAAGCGGAGGCTCTGG
CGGATCTGATGCCCTGGACGACTTCGACCTGGATATGCTGGGCAGCGACGCCCTGG
ATGATTTTGATCTGGACATGCTGGGATCTGACGCTCTGGACGATTTCGATCTCGACA
TGTTGGGATCAGATGCACTGGATGACTTTGACCTGGACATGCTCGGATCATGA
(SEQ ID NO: 5);

MALPVTALLLPLALLLHAARPEQKLISEEDLMADVQLVESGGGLVQAGGS
LRLSCAASGRTISMAAMSWFRQAPGKEREFVAGISRSAGSAVHADSVKGRFTISRDNT
KNTLYLQMNSLKAEDTAVYYCAVRTSGFFGSIPRTGTAFDYWGQGTQVTVSILDYSFT
GGAGRDIPPPQIEEACELPECQVDAGNKVCNLQCNNHACGWDGGDCSLNFNDPWKN

CTQSLQCWKYFSDGHCDSQCNSAGCLFDGFDCQLTEGQCNPLYDQYCKDHFSDGHC
DQGCNSAECEWDGLDCAEHVPERLAAGTLVLVVLLPPDQLRNNSFHFLRELSHVLHT
NVVFKRDAQGQQMIFPYYGHEEELRKHPIKRSTVGWATSSLLPGTSGGRQRRELDPM
DIRGSIVYLEIDNRQCVQSSSQCFQSATDVAAFLGALASLGSLNIPYKIEAVKSEPVEPP
LPSQLHLMYVAAAAFVLLFFVGCGVLLSRKRRRMKLLSSIEQACDICRLKKLKCSKE
KPKCAKCLKNNWECRYSPKTKRSPLTRAHLTEVESRLERLEQLFLLIFPREDLDMILK
MDSLQDIKALLTGLFVQDNVNKDAVTDRLASVETDMPLTLRQHRISATSSSEESSNKG
QRQLTVSAAAGGSGGSGGSDALDDFDLDMLGSDALDDFDLDMLGSDALDDFDLDM
LGSDALDDFDLDMLGS (SEQ ID NO: 6);

ATGGCATTGCCCGTGACCGCCCTGCTGCTGCCACTGGCCTTGTTGCTCC
ACGCCGCGCGGCCAGAACAGAAGCTGATCAGCGAGGAGGATCTGATGGCTGATGT
GCAACTCGTAGAGTCCGGTGGCGGACTCGTCCAGGCGGGAGGCTCCCTGCGCCTG
AGTTGCGCTGCGTCCGGAAGAACTATTAGCATGGCCGCCATGAGTTGGTTCAGACA
GGCGCCTGGAAAGGAGAGGGAGTTCGTGGCAGGCATAAGTCGCTCCGCAGGGTC
AGCCGTCCATGCCGACTCCGTAAAGGGACGCTTTACCATCTCCAGAGATAACACAA
AAAACACGCTCTATCTCCAAATGAATTCTTTGAAGGCCGAGGACACAGCCGTGTAC
TACTGCGCCGTTAGGACCAGCGGATTTTTTGGAAGCATTCCTAGGACAGGGACAG
CCTTTGATTACTGGGGCCAGGGAACGCAGGTTACCGTTTCCATCCTGGACTACAGC
TTCACAGGTGGCGCTGGGCGCGACATTCCCCCACCGCAGATTGAGGAGGCCTGTG
AGCTGCCTGAGTGCCAGGTGGATGCAGGCAATAAGGTCTGCAACCTGCAGTGTAA
TAATCACGCATGTGGCTGGGATGGTGGCGACTGCTCCCTCAACTTCAATGACCCCT
GGAAGAACTGCACGCAGTCTCTACAGTGCTGGAAGTATTTTAGCGACGGCCACTG
TGACAGCCAGTGCAACTCGGCCGGCTGCCTCTTTGATGGCTTCGACTGCCAGCTC
ACCGAGGGACAGTGCAACCCCCTGTATGACCAGTACTGCAAGGACCACTTCAGTG
ATGGCCACTGCGACCAGGGCTGTAACAGTGCCGAATGTGAGTGGGATGGCCTAGA
CTGTGCTGAGCATGTACCCGAGCGGCTGGCAGCCGGCACCCTGGTGCTGGTGGTG
CTGCTTCCACCCGACCAGCTACGGAACAACTCCTTCCACTTTCTGCGGGAGCTCA
GCCACGTGCTGCACACCAACGTGGTCTTCAAGCGTGATGCGCAAGGCCAGCAGAT
GATCTTCCCGTACTATGGCCACGAGGAAGAGCTGCGCAAGCACCCAATCAAGCGC
TCTACAGTGGGTTGGGCCACCTCTTCACTGCTTCCTGGTACCAGTGGTGGGCGCCA

GCGCAGGGAGCTGGACCCCATGGACATCCGTGGCTCCATTGTCTACCTGGAGATCG
ACAACCGGCAATGTGTGCAGTCATCCTCGCAGTGCTTCCAGAGTGCCACCGATGT
GGCTGCCTTCCTAGGTGCTCTTGCGTCACTTGGCAGCCTCAATATTCCTTACAAGAT
TGAGGCCGTGAAGAGTGAGCCGGTGGAGCCTCCGCTGCCCTCGCAGCTGCACCTC
ATGTACGTGGCAGCGGCCGCCTTCGTGCTCCTGTTCTTTGTGGGCTGTGGGGTGCT
GCTGTCCCGCAAGCGCCGGCGGATGAAGCTGCTGAGCAGCATCGAGCAGGCCTGT
GACATCTGCCGGCTGAAGAAACTGAAGTGCAGCAAAGAAAAGCCCAAGTGCGCC
AAGTGCCTGAAGAACAACTGGGAGTGCCGGTACAGCCCCAAGACCAAGAGAAGC
CCCCTGACCAGAGCCCACCTGACCGAGGTGGAAAGCCGGCTGGAAAGACTGGAA
CAGCTGTTTCTGCTGATCTTCCCACGCGAGGACCTGGACATGATCCTGAAGATGGA
CAGCCTGCAGGACATCAAGGCCCTGCTGACCGGCCTGTTCGTGCAGGACAACGTG
AACAAGGACGCCGTGACCGACAGACTGGCCAGCGTGGAAACCGACATGCCCCTG
ACCCTGCGGCAGCACAGAATCAGCGCCACCAGCAGCAGCGAGGAAAGCAGCAAC
AAGGGCCAGCGGCAGCTGACAGTGTCTGCTGCTGCAGGCGGAAGCGGAGGCTCT
GGCGGATCTGATGCCCTGGACGACTTCGACCTGGATATGCTGGGCAGCGACGCCCT
GGATGATTTTGATCTGGACATGCTGGGATCTGACGCTCTGGACGATTTCGATCTCGA
CATGTTGGGATCAGATGCACTGGATGACTTTGACCTGGACATGCTCGGATCATGA
(SEQ ID NO: 7).

### Example 2: Testing and verification of SynNotch receptors at the cellular level

In this Example, the specific process of SynNotch receptor cells recognizing specific extracellular signals is as follows: when the anti-GFP antibody single chain in the extracellular domain of the SynNotch receptor bonds to the GFP antigen of the sender cell, the Notch transmembrane domain is cleaved by the protease, and GAL4VP64 is released into the cell. GAL4 can specifically recognize and bind to the GAL4 binding site (UAS region), and the VP64 transcription activation element will activate the transcription of the downstream BFP gene, promoting the expression of the BFP fluorescent protein. See FIG 2 for details.

In the present disclosure, HT1080 cells were used as sender cells, and another batch of HT1080 cells were used as receiver cells to verify the ability of the expression plasmid vector (pCDH-antiGFP-XENTR-SynNotch-Gal4VP64) prepared in Example 1 to enhance the transcription and activation of downstream genes at the cellular level. The specific verification steps were as follows.
(1) HT1080 cell lines were infected with lentivirus, which can stably express membrane-anchored GFP and serve as sender cells (the structure of membrane-anchored GFP was shown in FIG. 3, and GFP has an amino acid sequence as set forth in SEQ ID NO: 8 and a nucleotide sequence as set forth in SEQ ID NO: 9).

The amino acid sequence and nucleotide sequence of GFP were as follows: 
METDTLLLWVLLLWVPGSTGDMVSKGEELFTGVVPILVELDGDVNGHKFS
VSGEGEGDATYGKLTLKFICTTGKLPVPWPTLVTTLTYGVQCFSRYPDHMKQHDFFKS
AMPEGYVQERTIFFKDDGNYKTRAEVKFEGDTLVNRIELKGIDFKEDGNILGHKLEYN
YNSHNVYIMADKQKNGIKVNFKIRHNIEDGSVQLADHYQQNTPIGDGPVLLPDNHYL
STQSALSKDPNEKRDHMVLLEFVTAAGITLGMDELYKNAVGQDTQEVIVVPHSLPFK
VVVISAILALVVLTIISLIILIMLWQKKPR (SEQIDNO:8);
 and

ATGGAGACCGACACCCTGCTGCTGTGGGTGCTGCTGCTGTGGGTGCCCG
GCAGCACCGGCGACATGGTGAGCAAGGGCGAGGAGCTGTTCACCGGCGTGGTGC
CCATCCTGGTGGAGCTGGACGGCGACGTGAACGGCCACAAGTTCAGCGTGAGCG
GCGAGGGCGAGGGCGACGCCACCTACGGCAAGCTGACCCTGAAGTTCATCTGCAC
CACCGGCAAGCTGCCCGTGCCCTGGCCCACCCTGGTGACCACCCTGACCTACGGC
GTGCAGTGCTTCAGCCGCTACCCCGACCACATGAAGCAGCACGACTTCTTCAAGA
GCGCCATGCCCGAGGGCTACGTGCAGGAGCGCACCATCTTCTTCAAGGACGACGG
CAACTACAAGACCCGCGCCGAGGTGAAGTTCGAGGGCGACACCCTGGTGAACCG
CATCGAGCTGAAGGGCATCGACTTCAAGGAGGACGGCAACATCCTGGGCCACAAG
CTGGAGTACAACTACAACAGCCACAACGTGTACATCATGGCCGACAAGCAGAAGA
ACGGCATCAAGGTGAACTTCAAGATCCGCCACAACATCGAGGACGGCAGCGTGCA
GCTGGCCGACCACTACCAGCAGAACACCCCCATCGGCGACGGCCCCGTGCTGCTG
CCCGACAACCACTACCTGAGCACCCAGAGCGCCCTGAGCAAGGACCCCAACGAG
AAGCGCGACCACATGGTGCTGCTGGAGTTCGTGACCGCCGCCGGCATCACCCTGG
GCATGGACGAGCTGTACAAGAACGCCGTGGGCCAGGACACCCAGGAGGTGATCG
TGGTGCCCCACAGCCTGCCCTTCAAGGTGGTGGTGATCAGCGCCATCCTGGCCCTG
GTGGTGCTGACCATCATCAGCCTGATCATCCTGATCATGCTGTGGCAGAAGAAGCC
CCGC (SEQ ID NO:9).

Another batch of HT1080 cells were taken as receiver cells, which were first infected with pHR_Gal4UAS_tBFP_PGK_mCherry lentivirus (purchased from Addgene, #79130, after infecting the host cells, the lentivirus can be induced by GAL4VP64 to express BFP fluorescent protein). The cells that can stably and highly express pHR_Gal4UAS_tBFP_PGK_mCherry were sorted out, and then infected with pCDH-antiGFP-XENTR-SynNotch-Gal4VP64 lentivirus.

The pHR_Gal4UAS_tBFP_PGK_mCherry and pCDH-antiGFP-XENTR-SynNotch-Gal4VP64 lentiviruses were obtained by co-transfecting the expression plasmid (pCDH-antiGFP-XENTR-SynNotch-Gal4VP64 or pHR_Gal4UAS_tBFP_PGK_mCherry), pMD2.G, and psPAX2 into a 15 cm culture dish containing HEK293-T cells at a density of 90%, and collecting the cell supernatant three days later, filtering the virus solution using a 0.45 µM filter, and concentrating the virus.

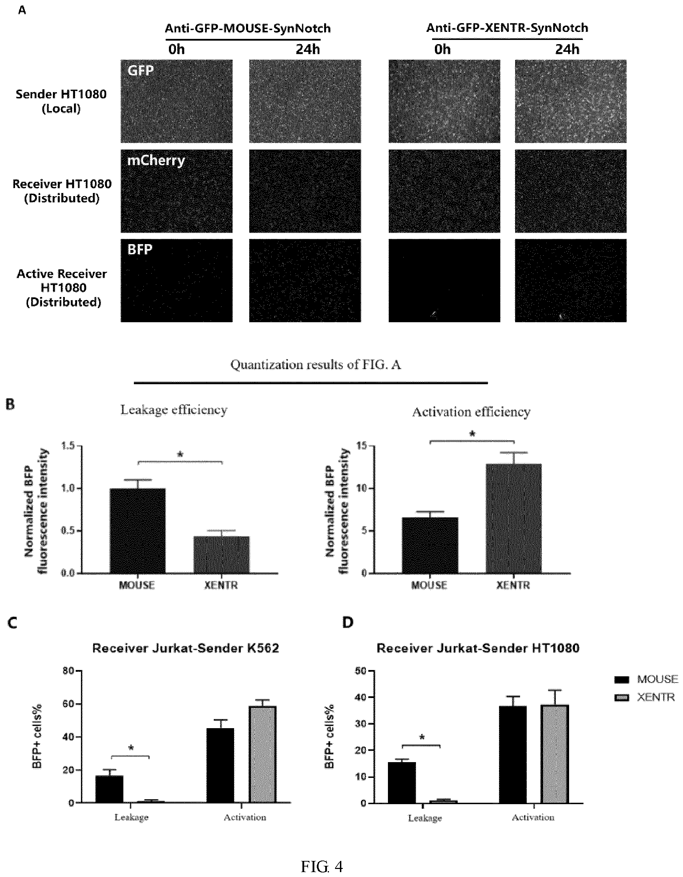

(2) The above-mentioned stably transfected receiver cell HT1080 cells were taken and inoculated into a 24-well plate at a cell density of 3×10⁵ cells/mL, and HT1080 cells expressing GFP at a cell density of 3×10⁵ cells/mL were added and co-cultured for 48 hours. The cell photos were taken by using an Olympus inverted fluorescence microscope, and the fluorescence intensity expressed by the cells was analyzed by using ImageJ. The results were shown in FIG 4.

FIG 4A shows a comparison of fluorescence microscope photos, in which MOUSE-Syn represents the SynNotch synthetic receptor containing the mouse Notch transmembrane domain (abbreviated as SynNotch synthetic receptor 2), which is the original system; XENTR-Syn represents the SynNotch synthetic receptor containing the *Xenopus tropicalis* Notch transmembrane domain (abbreviated as SynNotch synthetic receptor 1). As shown in FIG 4A, compared with SynNotch synthetic receptor 2, SynNotch synthetic receptor 1 can significantly improve the activation efficiency.

FIG 4B shows the average fluorescence intensity of BFP blue fluorescent protein expressed in HT1080 cells analyzed by ImageJ software. As shown in FIG 4B, compared with SynNotch synthetic receptor 2 (i.e., MOUSE group), the activation efficiency of SynNotch synthetic receptor 1 (i.e., XENTR group) was increased by 95.1%, and the leakage level of SynNotch synthetic receptor 1 (i.e., XENTR group) was reduced by 56.4%.

Since there are three forms of cell-to-cell contact, including contact between adherent cells, contact between adherent cells and suspended cells, and contact between suspended cells. The three contact forms also reflect to a certain extent the three real contact environments of cells in vivo. Therefore, it is very important to determine whether all the three cell contact forms can effectively activate the SynNotch system. HT1080 is an adherent cell, and the above results in FIG 4A and FIG 4B can only prove that the contact between adherent cells can activate the SynNotch system, but it cannot be determined for the other two contact systems. Therefore, the suspended receiver cells Jurkat and the suspended sender cells K562 were further constructed according to step (1) of Example 2. Further, according to step (2) of Example 2, the sender cells K562 and the receiver cells Jurkat were co-cultured for 48 hours to observe whether the contact between the suspended cells could activate the SynNotch system; at the same time, the sender cells HT1080 and the receiver cells Jurkat were co-cultured for 48 hours to observe whether the contact between the suspended cells and the adherent cells could activate the SynNotch system. The results were shown in FIG 4C and FIG 4D, XENTR-SynNotch and MOUSE-SynNotch can be effectively activated in the contact between suspended receiver cells and suspended sender cells or in the contact between suspended receiver cells and adherent sender cells. Moreover, the leakage activation efficiency of XENTR-SynNotch was much lower than that of MOUSE-SynNotch, and the effective activation efficiency was slightly higher than that of MOUSE-SynNotch.

Therefore, it can be illustrated that the SynNotch system containing the transmembrane region of the Notch receptor protein derived from *Xenopus tropicalis* designed by the present disclosure has a stronger activation efficiency than the original SynNotch receptor system containing the transmembrane region of the Notch receptor protein derived from mouse.

### Example 3: Activation of SynNotch-CAR-gated chimeric antigen receptor-combined Jurkat T cells by target cancer cells

Currently, T cells expressing chimeric antigen receptors (CAR) are effective as a treatment for certain B-cell cancers. However, a major concern of CAR-T cell cancer immunotherapy is off-target effects, in which therapeutic CAR-T cells will destroy normal tissues, leading to severe side effects or even death. A possible strategy to alleviate such problems is to make therapeutic CAR-T cells have more precise tumor recognition targets. Moreover, adding a tumor antigen recognition can enable CAR express and thus provide a more precise T cell response, which is an effective means. To implement this strategy, it was inferred that the SynNotch synthetic receptor could be used in the design of therapeutic CAR-T cells, that is, firstly, the tumor is detected by binding to a tumor-specific cell surface antigen and CAR expression was only triggered in response to a second tumor-specific antigen in the tumor, thereby providing both dual antigen control of CAR-T cell activity and a tumor-localized response.

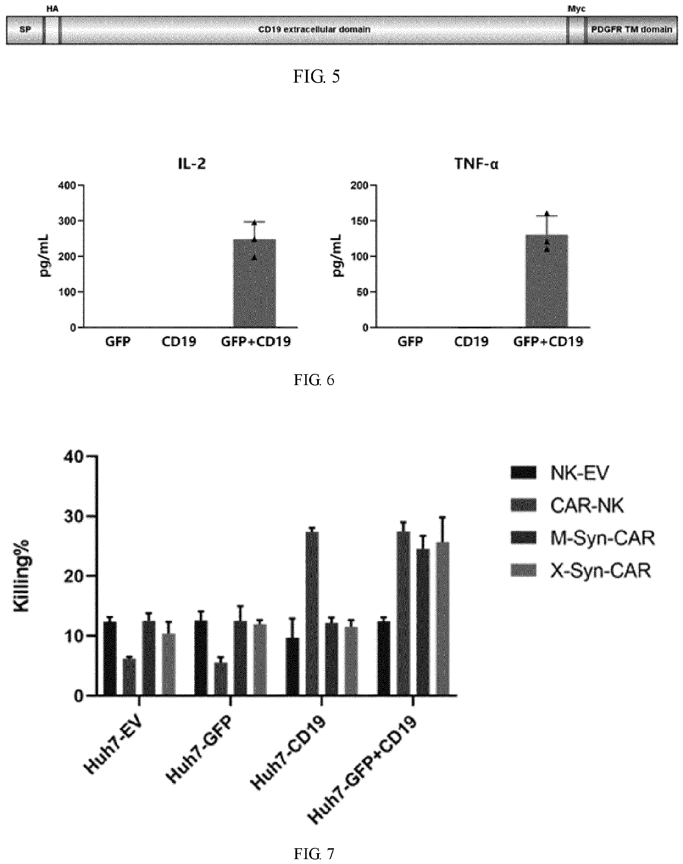

Based on this, the inventors designed SynNotch-CAR-gated chimeric antigen receptor-combined Jurkat cells (abbreviated as SynNotch-CAR-Jurkat T cells) including the corresponding experiments performed in Jurkat cells (T cell line), specifically including the following steps:
(1) The SynNotch synthetic receptor 1 in Example 2 was packaged with a lentivirus, and the lentivirus packaging can recognize the CAR molecule of CD19. 5'-end of the nucleic acid encoding the CAR molecule included a UAS-minimal-CMV sequence, which can bind to the intracellular polypeptide produced after the activation of the SynNotch synthetic receptor to induce the expression of the CAR molecule (the SynNotch synthetic receptor 1 was directly synthesized and lentivirus-packaged by GENEWIZ, Inc., the CAR molecule has an amino acid sequence as set forth in SEQ ID NO: 10 and a nucleotide sequence as set forth in SEQ ID NO: 11, and the CAR molecule was hereinafter referred to as an inducible CAR molecule). The above-mentioned two viruses were used to infect Jurkat T cells at the same time, and Jurkat T cells that stably express the above-mentioned SynNotch synthetic receptor and inducible CAR molecule were obtained by screening;
(2) K562 cells expressing membrane-anchored GFP alone, K562 cells expressing membrane-anchored CD19 alone (the structure was shown in FIG 5, CD19 had an amino acid sequence as set forth in SEQ ID NO: 12 and a nucleotide sequence as set forth in SEQ ID NO: 13), and K562 cells co-expressing membrane-anchored GFP and CD19 were constructed as sender cells by conventional methods in the art; and
(3) The three types of K562 cells in step (2) were co-cultured with the Jurkat cells constructed in step (1), and the expression of CD69 and the secretion of IL-2 and TNF-α in the Jurkat cells of each group were detected after 24 hours. The results were shown in FIG 6, in which, the expression of CD69 and the secretion of IL-2 and TNF-α represent the activation of Jurkat T cells.

As shown in FIG 6, SynNotch-CAR-Jurkat T cells can be activated only when exposed to K562 expressing two antigens (GFP and CD19).

MALPVTALLLPLALLLHAARPEQKLISEEDLDIQMTQTTSSLSASLGDRVTI
SCRASQDISKYLNWYQQKPDGTVKLLIYHTSRLHSGVPSRFSGSGSGTDYSLTISNLE
QEDIATYFCQQGNTLPYTFGGGTKLEITGGGGSGGGGSGGGGSEVKLQESGPGLVAPS
QSLSVTCTVSGVSLPDYGVSWIRQPPRKGLEWLGVIWGSETTYYNSALKSRLTIIKDN
SKSQVFLKMNSLQTDDTAIYYCAKHYYYGGSYAMDYWGQGTSVTVSSKPTTTPAPR
PPTPAPTIASQPLSLRPEACRPAAGGAVHTRGLDFACDIYIWAPLAGTCGVLLLSLVITK
RGRKKLLYIFKQPFMRPVQTTQEEDGCSCRFPEEEEGGCELRVKFSRSADAPAYQQGQ
NQLYNELNLGRREEYDVLDKRRGRDPEMGGKPQRRKNPQEGLYNELQKDKMAEAY
SEIGMKGERRRGKGHDGLYQGLSTATKDTYDALHMQALPPR (SEQ ID NO: 10);

GGAGCACTGTCCTCCGAACGTCGGAGCACTGTCCTCCGAACGTCGGAG
CACTGTCCTCCGAACGTCGGAGCACTGTCCTCCGAACGGAGCATGTCCTCCGAAC
GTCGGAGCACTGTCCTCCGAACGACTAGTTAGGCGTGTACGGTGGGAGGCCTATAT
AAGCAGAGCTCGTTTAGTGAACCGTCAGATCGCCTGGAGACGCCATCCACGCTGT
TTTGACCTCCATAGAAGACACCGGGACCGATCCAGCCTCTCGACATTCGTTGGATC
CATGGCATTGCCCGTGACCGCCCTGCTGCTGCCACTGGCCTTGTTGCTCCACGCCG
CGCGGCCAGAACAGAAGCTGATCAGCGAGGAGGATCTGGATATACAGATGACGCA
GACAACGTCAAGTCTTTCCGCCAGCTTGGGAGACCGAGTGACTATATCTTGTAGAG
CAAGCCAGGATATTTCTAAGTATCTTAACTGGTACCAACAAAAGCCCGATGGAACG
GTTAAGCTGCTTATATACCATACCAGTAGACTCCACTCCGGCGTACCATCACGGTTT
TCTGGCAGTGGCTCCGGGACCGACTATTCTTTGACGATCTCTAATCTCGAACAAGA
GGATATTGCAACATACTTTTGTCAGCAAGGCAATACCTTGCCATATACGTTTGGGGG
CGGGACAAAACTTGAGATAACCGGCGGCGGTGGTTCAGGCGGTGGCGGTTCCGGT
GGTGGGGGATCAGAGGTTAAGCTTCAGGAATCCGGACCAGGTTTGGTTGCCCCCA
GCCAATCTCTCAGCGTTACATGCACGGTTTCAGGCGTCAGTCTCCCCGATTACGGT
GTAAGTTGGATTCGGCAACCTCCGCGAAAGGGTCTGGAATGGCTGGGGGTTATTTG

GGGGAGTGAGACAACTTATTACAACTCTGCACTTAAGAGTCGGCTTACCATCATCA
AGGATAATTCAAAATCACAAGTATTCCTGAAGATGAACTCATTGCAAACAGATGAT
ACAGCTATATACTATTGTGCCAAGCATTACTATTATGGTGGTTCTTATGCAATGGATT
ACTGGGGGCAAGGCACGTCAGTGACAGTGAGTTCAAAGCCCACCACGACGCCAG
CGCCGCGACCACCAACACCGGCGCCCACCATCGCGTCGCAGCCCCTGTCCCTGCG
CCCAGAGGCGTGCCGGCCAGCGGCGGGGGGCGCAGTGCACACGAGGGGGCTGGA
CTTCGCCTGTGATATCTACATCTGGGCGCCCTTGGCCGGGACTTGTGGGGTCCTTCT
CCTGTCACTGGTTATCACCAAACGGGGCAGAAAGAAACTCCTGTATATATTCAAAC
AACCATTTATGAGACCAGTACAAACTACTCAAGAGGAAGATGGCTGTAGCTGCCGA
TTTCCAGAAGAAGAAGAAGGAGGATGTGAACTGAGAGTGAAGTTCAGCAGGAGC
GCAGACGCCCCCGCGTACCAGCAGGGCCAGAACCAGCTCTATAACGAGCTCAATC
TAGGACGAAGAGAGGAGTACGATGTTTTGGACAAGAGACGTGGCCGGGACCCTG
AGATGGGGGGAAAGCCGCAGAGAAGGAAGAACCCTCAGGAAGGCCTGTACAATG
AACTGCAGAAAGATAAGATGGCGGAGGCCTACAGTGAGATTGGGATGAAAGGCG
AGCGCCGGAGGGGCAAGGGGCACGATGGCCTTTACCAGGGTCTCAGTACAGCCAC
CAAGGACACCTACGACGCCCTTCACATGCAGGCCCTGCCCCCTCGC (SEQ ID NO:
11);

METDTLLLWVLLLWVPGSTGDYPYDVPDYAGAQPARPEEPLVVKVEEGD
NAVLQCLKGTSDGPTQQLTWSRESPLKPFLKLSLGLPGLGIHMRPLAIWLFIFNVSQQ
MGGFYLCQPGPPSEKAWQPGWTVNVEGSGELFRWNVSDLGGLGCGLKNRSSEGPSS
PSGKLMSPKLYVWAKDRPEIWEGEPPCLPPRDSLNQSLSQDLTMAPGSTLWLSCGVPP
DSVSRGPLSWTHVHPKGPKSLLSLELKDDRPARDMWVMETGLLLPRATAQDAGKYY
CHRGNLTMSFHLEITARPVLWHWLLRTGGWKVDEQKLISEEDLNAVGQDTQEVIVVP
HSLPFKVVVISAILALVVLTIISLIILIMLWQKKPR (SEQIDNO:12); and

ATGGAGACCGACACCCTGCTGCTGTGGGTGCTGCTGCTGTGGGTGCCCG
GCAGCACCGGCGACTACCCCTACGACGTGCCCGACTACGCCGGCGCCCAGCCCGC
CCGCCCCGAGGAGCCCCTGGTGGTGAAGGTGGAGGAGGGCGACAACGCCGTGCT
GCAGTGCCTGAAGGGCACCAGCGACGGCCCCACCCAGCAGCTGACCTGGAGCCG
CGAGAGCCCCCTGAAGCCCTTCCTGAAGCTGAGCCTGGGCCTGCCCGGCCTGGGC
ATCCACATGCGCCCCCTGGCCATCTGGCTGTTCATCTTCAACGTGAGCCAGCAGAT

GGGCGGCTTCTACCTGTGCCAGCCCGGCCCCCCCAGCGAGAAGGCCTGGCAGCCC
GGCTGGACCGTGAACGTGGAGGGCAGCGGCGAGCTGTTCCGCTGGAACGTGAGC
GACCTGGGCGGCCTGGGCTGCGGCCTGAAGAACCGCAGCAGCGAGGGCCCCAGC
AGCCCCAGCGGCAAGCTGATGAGCCCCAAGCTGTACGTGTGGGCCAAGGACCGC
CCCGAGATCTGGGAGGGCGAGCCCCCCTGCCTGCCCCCCCGCGACAGCCTGAACC
AGAGCCTGAGCCAGGACCTGACCATGGCCCCCGGCAGCACCCTGTGGCTGAGCTG
CGGCGTGCCCCCCGACAGCGTGAGCCGCGGCCCCCTGAGCTGGACCCACGTGCAC
CCCAAGGGCCCCAAGAGCCTGCTGAGCCTGGAGCTGAAGGACGACCGCCCCGCC
CGCGACATGTGGGTGATGGAGACCGGCCTGCTGCTGCCCCGCGCCACCGCCCAGG
ACGCCGGCAAGTACTACTGCCACCGCGGCAACCTGACCATGAGCTTCCACCTGGA
GATCACCGCCCGCCCCGTGCTGTGGCACTGGCTGCTGCGCACCGGCGGCTGGAAG
GTGGACGAGCAGAAGCTGATCAGCGAGGAGGACCTGAACGCCGTGGGCCAGGAC
ACCCAGGAGGTGATCGTGGTGCCCCACAGCCTGCCCTTCAAGGTGGTGGTGATCA
GCGCCATCCTGGCCCTGGTGGTGCTGACCATCATCAGCCTGATCATCCTGATCATGC
TGTGGCAGAAGAAGCCCCGC (SEQ ID NO: 13).

### Example 4: Killing of target cancer cells by SynNotch-CAR-gated chimeric antigen receptor-combined NK cells (referred to as SynNotch-CAR-NK cells)

The SynNotch synthetic receptor 1 according to the present disclosure can not only play a role in T cells, but should also be universally applicable in other types of immune cells. By applying the same SynNotch-CAR system as described in Example 4 in NK cells, the inventors confirmed that it can also play a role in other immune cells, specifically including the following steps:
(1) The SynNotch synthetic receptor (SynNotch synthetic receptor 1 or SynNotch synthetic receptor 2) in Example 2 was packaged with a lentivirus, and the lentivirus packaging can recognize the CAR molecule of CD19. 5'-end of the nucleic acid sequence encoding the CAR molecule included a UAS-minimal-CMV sequence, which can bind to the intracellular polypeptide produced after the activation of the SynNotch synthetic receptor to induce the expression of the CAR molecule (the SynNotch synthetic receptor was directly synthesized and lentivirus-packaged by GENEWIZ, Inc., the CAR molecule had an amino acid sequence as set forth in SEQ ID NO: 10 and a nucleotide sequence as set forth in SEQ ID NO: 11). The above-mentioned two viruses were used to infect NK cells at the same time, and NK cells that stably express the above-mentioned SynNotch synthetic receptors and CAR molecules were obtained by screening; at the same time, NK cells that stably express CAR molecules that can recognize CD19 (i.e., CAR-NK) were set as a positive killing control group, and NK-EV (NK cells infected with viruses produced by empty plasmids) were set as a negative killing control group;
(2) Huh7 cells expressing membrane-anchored GFP alone, Huh7 cells expressing membrane-anchored CD19 alone (for specific sequences, see step (2) of Example 3), and Huh7 cells co-expressing membrane-anchored GFP and CD19 were constructed as sender cells;
(3) The sender cells described in (2) were labelled with CTV dye (purchased from Thermo Fisher); and
(4) The three types of Huh7 cells in step (3) were co-cultured with the NK cells constructed in (1), and the cells were collected after 48 hours. Dead cells were labeled with PI dye, and the viability of Huh7 cells in each group was analyzed and detected. See FIG 7 for details, in which the death ratio of Huh7 cells represents the killing of NK cells on the sender cells.

As shown in FIG 7, when exposed to Huh7 cells that simultaneously express two antigens (GFP and CD19), SynNotch-CAR-NK cells can be significantly activated and exert a higher killing effect on Huh7 cells. Compared with the combination of SynNotch-CAR (M-Syn-CAR) containing SynNotch synthetic receptor 2, the killing efficiency of SynNotch-CAR (X-Syn-CAR) containing SynNotch synthetic receptor 1 was slightly improved; and there was no significant difference in the killing efficiency of NK cells in combination with SynNotch-CAR containing SynNotch synthetic receptor 1 compared with NK cells that stably expressed CAR molecules (CAR-NK) that can recognize CD19.

In the description of this specification, the descriptions referring to the terms such as "one embodiment", "some embodiments", "example", "specific example", or "some examples" mean that the specific features, structures, materials, or characteristics described in connection with this embodiment or example are included in at least an embodiment or example of the present disclosure. In this specification, the exemplary descriptions of the foregoing terms do not necessarily refer to the same embodiment or example. Moreover, the specific features, structures, materials, or characteristics described may be combined in any one or more embodiments or examples in a suitable manner. Furthermore, those skilled in the art may combine and integrate different embodiments or examples and features of different embodiments or examples described in this specification without contradicting each other.

Although the embodiments of the present disclosure are illustrated and described above, it should be understood that the above embodiments are exemplary and should not be construed as limitations of the present disclosure. Those skilled in the art can make changes, modifications, substitutions, and variations to the above embodiments within the scope of the present disclosure.

## Claims

1. Use of an isolated polypeptide in the preparation of a SynNotch synthetic receptor, wherein the isolated polypeptide has at least 80% identity with a transmembrane region of a Notch receptor protein derived from *Xenopus tropicalis.*

2. A chimeric polypeptide, comprising:
an extracellular region having an activity of binding to a first molecule;
a transmembrane region, an N-terminus of the transmembrane region being linked to a C-terminus of the extracellular region; and
an intracellular region, an N-terminus of the intracellular region being linked to a C-terminus of the transmembrane region,
wherein the transmembrane region comprises an isolated polypeptide, the isolated polypeptide having at least 80% identity with a transmembrane region of a Notch receptor protein derived from *Xenopus tropicalis.*

3. The use according to claim 1 or the chimeric polypeptide according to claim 2, wherein the isolated polypeptide has 100% identity with the transmembrane region of the Notch receptor protein derived from *Xenopus tropicalis.*

4. The use according to claim 1 or the chimeric polypeptide according to claim 2, wherein the transmembrane region of the Notch receptor protein derived from *Xenopus tropicalis* has an amino acid sequence as set forth in SEQ ID NO: 1.

5. The chimeric polypeptide according to claim 2, wherein the first molecule comprises at least one of a tumor antigen, a virus, a bacterium, an endotoxin, an antibody, a cell receptor, and a ligand of a cell receptor.

6. The chimeric polypeptide according to claim 5, wherein the tumor antigen comprises at least one of a tumor-associated antigen and a tumor-specific antigen; and preferably, the tumor antigen is a tumor-specific antigen.

7. The chimeric polypeptide according to claim 2, wherein the first molecule comprises but is not limited to at least one of GFP, eGFP, CD19, ALPPL2, BCMA, SIRPα, CD1a, CD1b, CD1c, CD1d, CD1e, CD2, CD3d, CD3e, CD3g, CD4, CD5, CD7, CD8a, CD8b, CD20, CD21, CD22, CD23, CD25, CD27, CD28, CD30, CD33, CD34, CD38, CD40, CD44, CD44v6, CD45, CD48, CD51, CD52, CD56, CD59, CD66, CD70, CD71, CD72, CD73, CD74, CD79A, CD79B, CD80, CD86, CD94, CD95, CD133, CD134, CD140, CD152, CD154, CD158, CD178, CD181, CD182, CD183, CD200, CD210, CD221, CD246, CD252, CD253, CD261, CD262, CD273, CD274, CD276, CD279, CD295, CD339, CD340, EGFR, HER2, FGFR2, AFP, CA125, MSLN, GPC3, CEA, CLDN18.2, EpCAM, PSCA, GD2, IL-13, IL-13RA2, ROR1, MUC 1, PSMA, MAGEA1, 4-1BB, 5T4, BAFF, CA242, CA-IX, MET, CCR4, CNTO888, FAP, MORAb-009, VEGF-A, VEGFR-1, and VEGFR-2.

8. The chimeric polypeptide according to claim 2, wherein the extracellular region comprises a first binding protein or a fragment thereof that binds to the first molecule.

9. The chimeric polypeptide according to claim 8, wherein the first binding protein or the fragment thereof comprises at least one of an antibody or a functional fragment thereof, a receptor, a ligand of a receptor, and a cell adhesion molecule.

10. The chimeric polypeptide according to claim 8, wherein the first binding protein or the fragment thereof is a monoclonal antibody or a polyclonal antibody that binds to the first molecule.

11. The chimeric polypeptide according to claim 10, wherein the monoclonal antibody comprises at least one of a Fab antibody, a F(ab')₂ fragment, a Fv antibody, a single-chain antibody, a single-domain antibody, and a minimal recognition unit.

12. The chimeric polypeptide according to claim 2, wherein the intracellular region comprises at least one of a transcription activator protein, a transcription repressor protein, a transcription factor, a site-specific nuclease, a recombinase, an activating immune receptor intracellular domain, and an inhibitory immune receptor intracellular domain.

13. The chimeric polypeptide according to claim 12, wherein the intracellular region comprises at least one of GaL4 VP64, GaL4 VP16, tetR VP64, ZFHD1 VP64, Gal4 KRAB, HAP1 VP16, LexA VP64, Cas9, and Cas13.

14. The chimeric polypeptide according to claim 13, wherein the Gal4 VP64 has an amino acid sequence as set forth in SEQ ID NO: 2.

15. The chimeric polypeptide according to claim 2, wherein:
the transmembrane region and the intracellular region have an amino acid sequence as set forth in SEQ ID NO: 3; and/or
the chimeric polypeptide has an amino acid sequence as set forth in SEQ ID NO: 4.

16. A first nucleic acid molecule, encoding the chimeric polypeptide according to any one of claims 2 to 15, wherein the first nucleic acid molecule is DNA.

17. A first expression vector, carrying the first nucleic acid molecule according to claim 16.

18. The first expression vector according to claim 17, wherein the first expression vector is a eukaryotic expression vector, a prokaryotic expression vector, a virus, or a bacteriophage; and preferably, a plasmid expression vector.

19. A recombinant cell, carrying the first nucleic acid molecule according to claim 16 or the first expression vector according to any one of claims 17 to 18; or expressing the chimeric polypeptide according to any one of claims 2 to 15.

20. The recombinant cell according to claim 19, wherein:
the recombinant cell is obtained by introducing the first expression vector according to any one of claims 17 to 18 into a host cell; and the host cell comprises at least one of an immune cell, a neuron, a progenitor cell or a precursor cell, an epithelial cell, an endothelial cell, and a stem cell.

21. The recombinant cell according to claim 20, wherein the host cell comprises at least one of a T cell, a B cell, a monocyte, a NK cell, a dendritic cell, a macrophage, a regulatory T cell, a helper T cell, a cytotoxic T cell, a NKT cell, and a γδ T cell.

22. The recombinant cell according to claim 19, wherein the recombinant cell further comprises a second nucleic acid molecule encoding a chimeric antigen receptor, a NK cell receptor, or a T cell receptor, or a second expression vector carrying the second nucleic acid molecule; or
wherein the recombinant cell expresses the chimeric antigen receptor, the NK cell receptor, or the T cell receptor.

23. The recombinant cell according to claim 22, wherein:
the intracellular region of the chimeric polypeptide comprises a transcription activator protein; and
5'-end of the second nucleic acid molecule encoding the chimeric antigen receptor, the NK cell receptor, or the T cell receptor is linked to an inducible expression nucleic acid sequence for binding to the transcription activator protein.

24. A pharmaceutical composition, comprising:
the chimeric polypeptide according to any one of claims 2 to 15, the first nucleic acid molecule according to claim 16, the first expression vector according to any one of claims 17 to 18, or the recombinant cell according to any one of claims 19 to 23; and
optionally, a pharmaceutically acceptable excipient.

25. Use of the chimeric polypeptide according to any one of claims 2 to 15, the first nucleic acid molecule according to claim 16, the first expression vector according to any one of claims 17 to 18, the recombinant cell according to any one of claims 19 to 23, or the pharmaceutical composition according to claim 24 in the preparation of a medicament for preventing and/or treating a disease; and
optionally, the disease comprises a cancer or a tumor, an autoimmune disease, an inflammation, and a related disease caused by cellular senescence.

26. Use of the chimeric polypeptide according to any one of claims 2 to 15, the first nucleic acid molecule according to claim 16, the first expression vector according to any one of claims 17 to 18, the recombinant cell according to any one of claims 19 to 23, or the pharmaceutical composition according to claim 24 in the prevention and/or treatment of a disease;
optionally, the disease comprises a cancer or a tumor, an autoimmune disease, an inflammation, and a related disease caused by cellular senescence.

27. A method for preventing and/or treating a disease, comprising:
administering a pharmaceutically acceptable dose of the chimeric polypeptide according to any one of claims 2 to 15, the first nucleic acid molecule according to claim 16, the first expression vector according to any one of claims 17 to 18, the recombinant cell according to any one of claims 19 to 23, or the pharmaceutical composition according to claim 24 to a subject,
optionally, wherein the disease comprises a cancer or a tumor, an autoimmune disease, an inflammation, and a related disease caused by cellular senescence.

28. A method for activating an immune cell, comprising:
performing a first contact on the immune cell and a first molecule, wherein the immune cell expresses the chimeric polypeptide according to any one of claims 2 to 15.

29. The method according to claim 28, wherein:
the intracellular region of the chimeric polypeptide comprises a transcription activator protein; the immune cell expresses a chimeric antigen receptor, a NK cell receptor, or a T cell receptor, the chimeric antigen receptor, the NK cell receptor, or the T cell receptor comprising an antibody or a functional fragment thereof that binds to a predetermined antigen; and
5'-end of a second nucleic acid molecule encoding the chimeric antigen receptor, the NK cell receptor, or the T cell receptor is linked to an inducible expression nucleic acid sequence for binding to the transcription activator protein; and
wherein the method further comprises, subsequent to the first contact:
releasing, by the immune cell, the transcription activator protein, and expressing, by the immune cell, the chimeric antigen receptor, the NK cell receptor, or the T cell receptor; and
activating the immune cell by binding the chimeric antigen receptor, the NK cell receptor, or the T cell receptor of the immune cell to the predetermined antigen,
optionally, wherein the immune cell comprises at least one of a T cell, a B cell, a monocyte, a NK cell, a dendritic cell, a macrophage, a regulatory T cell, a helper T cell, a cytotoxic T cell, a NKT cell, and a γδ T cell.

30. A method for tracking a first cell-second cell contact, comprising:
performing a second contact on the first cell and the second cell, wherein the first cell expresses the chimeric polypeptide according to any one of claims 2 to 15 and a reporter gene protein, the intracellular region of the chimeric polypeptide being a transcription activator protein, and 5'-end of a third nucleic acid molecule encoding the reporter gene protein being linked to an inducible expression nucleic acid sequence for binding to the transcription activator protein, and wherein the second cell expresses a first molecule; and
determining a contact situation between the first cell and the second cell based on a detection result of the reporter gene protein in the first cell.

31. The method according to claim 30, wherein:
the first cell or the second cell comprises at least one of an immune cell, a neuron, a progenitor cell or a precursor cell, an epithelial cell, an endothelial cell, and a stem cell; and
optionally, the first cell comprises at least one of a T cell, a B cell, a monocyte, a NK cell, a dendritic cell, a macrophage, a regulatory T cell, a helper T cell, a cytotoxic T cell, a NKT cell, and a γδ T cell.
